(19) Europäisches Patentamt
European Patent Office
Office européen des brevets

(11) EP 2 482 212 A1

(12) EUROPEAN PATENT APPLICATION
published in accordance with Art. 153(4) EPC

(43) Date of publication:
**01.08.2012 Bulletin 2012/31**

(21) Application number: **09849644.1**

(22) Date of filing: **25.09.2009**

(51) Int Cl.:
***G06F 17/50*** *(2006.01)* ***C07K 16/00*** *(2006.01)*
***C07K 14/00*** *(2006.01)*

(86) International application number:
**PCT/CN2009/001079**

(87) International publication number:
**WO 2011/035456 (31.03.2011 Gazette 2011/13)**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(71) Applicant: **Shanghai National Engineering Research Center
of Antibody Medicine Co., Ltd.
Zhangjiang Hi-Tech Park
Shanghai 201-203 (CN)**

(72) Inventors:
- **GUO, Yajun**
  **Shanghai 201203 (CN)**
- **LI, Bohua**
  **Shanghai 201203 (CN)**
- **WANG, Hao**
  **Shanghai 201203 (CN)**
- **HOU, Sheng**
  **Shanghai 201203 (CN)**
- **ZHAO, Lei**
  **Shanghai 201203 (CN)**

(74) Representative: **Høiberg A/S
St. Kongensgade 59 A
1264 Copenhagen K (DK)**

# (54) METHOD OF ACQUIRING PROTEINS WITH HIGH AFFINITY BY COMPUTER AIDED DESIGN

(57) The present invention provides a method of acquiring proteins with high affinity by computer-aided design, which comprises the steps of: 1) based on a known cocrystal structure of a complex of a protein and a target molecule, determining candidate mutation sites of the protein; 2) simulating amino acid mutations in candidate sites of the protein in turn by computer so as to acquire optimized structures; 3) searching out conformations of the optimized structures acquired in step 2) by computer; 4) analyzing the total energies and root mean square deviations of the conformations acquired in step 3), and then selecting conformations with minimized energy and less root mean square deviations to analyze binding energies binding to the target molecule and to acquire simulative structures; and 5) based on the simulative structures acquired in step 4), predicting and validating mutated proteins with high affinity.



Printed by Jouve, 75001 PARIS (FR)

SEQUENCE LISTING

<110> SHANGHAI NATIONAL ENGINEERING RESEARCH CENTER OF ANTIBODY MEDICINE CO.,LTD

<120> METHOD OF ACQUIRING PROTEINS WITH HIGH AFFINITY BY COMPUTER AIDED DESIGN

<130> 09P403761

<160> 50

<170> PatentIn version 3.5

<210> 1
<211> 990
<212> DNA
<213> Artificial sequence

<220>
<221> misc_feature
<222> (1)..(990)
<223> Nucleotide sequence of the heavy chain constant region of human antibody (CH)

<400> 1
gctagcacca agggcccatc ggtcttcccc ctggcaccct cctccaagag cacctctggg 60

ggcacagcgg ccctgggctg cctggtcaag gactacttcc ccgaaccggt gacggtgtcg 120

tggaactcag gcgccctgac cagcggcgtg cacaccttcc cggctgtcct acagtcctca 180

ggactctact ccctcagcag cgtggtgacc gtgccctcca gcagcttggg cacccagacc 240

tacatctgca acgtgaatca caagcccagc aacaccaagg tggacaagaa agttgagccc 300

aaatcttgtg acaaaactca cacatgccca ccgtgcccag cacctgaact cctgggggga 360

ccgtcagtct tcctcttccc cccaaaaccc aaggacaccc tcatgatctc ccggacccct 420

gaggtcacat gcgtggtggt ggacgtgagc cacgaagacc ctgaggtcaa gttcaactgg 480

tacgtggacg gcgtggaggt gcataatgcc aagacaaagc cgcgggaaga gcagtacaac 540

agcacgtacc gtgtggtcag cgtcctcacc gtcctgcacc aggactggct gaatggcaag 600

gagtacaagt gcaaggtctc caacaaagcc ctcccagccc ccatcgagaa aaccatctcc 660

aaagccaaag ggcagccccg agaaccacag gtgtacaccc tgcccccatc ccgggatgag 720

ctgaccaaga accaggtcag cctgacctgc ctggtcaaag gcttctatcc cagcgacatc 780

gccgtggagt gggagagcaa tgggcagccg gagaacaact acaagaccac gcctcccgtg 840

ctggactccg acggctcctt cttcctctac agcaagctca ccgtggacaa gagcaggtgg 900

cagcagggga acgtcttctc atgctccgtg atgcatgagg ctctgcacaa ccactacacg 960

cagaagagcc tctccctgtc tcccggtaaa 990

<210> 2
<211> 330
<212> PRT

<213> Artificial sequence

<220>
<221> MISC_FEATURE
<222> (1)..(330)
<223> Amino acid sequence of the heavy chain constant region of human antibody (CH)

<400> 2

```
Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Val Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
```

```
    210                 215                 220

Gln Pro Arg Glu Pro Gln Val Tyr Thr Leu Pro Pro Ser Arg Asp Glu
225                 230                 235                 240

Leu Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro Gly Lys
                325                 330


<210>  3
<211>  321
<212>  DNA
<213>  Artificial sequence


<220>
<221>  misc_feature
<222>  (1)..(321)
<223>  Nucleotide sequence of the  light chain constant region of human
antibody (CL)


<400>  3
cgtactgtgg ctgcaccatc tgtcttcatc ttcccgccat ctgatgagca gttgaaatct       60

ggaactgcct ctgttgtgtg cctgctgaat aacttctatc ccagagaggc caaagtacag      120

tggaaggtgg ataacgccct ccaatcgggt aactcccagg agagtgtcac agagcaggac      180

agcaaggaca gcacctacag cctcagcagc accctgacgc tgagcaaagc agactacgag      240

aaacacaaag tctacgcctg cgaagtcacc catcagggcc tgagctcgcc cgtcacaaag      300

agcttcaaca ggggagagtg t                                                321


<210>  4
<211>  107
<212>  PRT
<213>  Artificial sequence


<220>
```

<221> MISC_FEATURE
<222> (1)..(107)
<223> Amino acid sequence of the light chain constant region of human antibody (CL)

<400> 4

Arg Thr Val Ala Ala Pro Ser Val Phe Ile Phe Pro Pro Ser Asp Glu
1 5 10 15

Gln Leu Lys Ser Gly Thr Ala Ser Val Val Cys Leu Leu Asn Asn Phe
20 25 30

Tyr Pro Arg Glu Ala Lys Val Gln Trp Lys Val Asp Asn Ala Leu Gln
35 40 45

Ser Gly Asn Ser Gln Glu Ser Val Thr Glu Gln Asp Ser Lys Asp Ser
50 55 60

Thr Tyr Ser Leu Ser Ser Thr Leu Thr Leu Ser Lys Ala Asp Tyr Glu
65 70 75 80

Lys His Lys Val Tyr Ala Cys Glu Val Thr His Gln Gly Leu Ser Ser
85 90 95

Pro Val Thr Lys Ser Phe Asn Arg Gly Glu Cys
100 105

<210> 5
<211> 120
<212> PRT
<213> Artificial sequence

<220>
<221> MISC_FEATURE
<222> (1)..(120)
<223> Amino acid sequence of the heavy chain variable region of Hmut1 mutant

<400> 5

Glu Val Gln Leu Val Glu Ser Gly Gly Gly Leu Val Gln Pro Gly Gly
1 5 10 15

Ser Leu Arg Leu Ser Cys Ala Ala Ser Gly Phe Asn Ile Lys Asp Thr
20 25 30

Tyr Ile His Trp Val Arg Gln Ala Pro Gly Lys Gly Leu Glu Trp Val
35 40 45

Ala Arg Ile Tyr Pro Thr Asn Gly Tyr Thr Arg Tyr Ala Asp Ser Val
50 55 60

Lys Gly Arg Phe Thr Ile Ser Ala Asp Thr Ser Lys Asn Thr Ala Tyr
65                  70                  75                  80

Leu Gln Met Asn Ser Leu Arg Ala Glu Asp Thr Ala Val Tyr Tyr Cys
                85                  90                  95

Ser Arg Trp Gly Gly Asp Gly Phe Tyr Ala Met Asp Tyr Trp Gly Gln
            100                 105                 110

Gly Thr Leu Val Thr Val Ser Ser
        115                 120

<210> 6
<211> 107
<212> PRT
<213> Artificial sequence

<220>
<221> MISC_FEATURE
<222> (1)..(107)
<223> Amino acid sequence of the light chain variable region of Hmut1 mutant

<400> 6

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Arg Val Asn Thr Ala
            20                  25                  30

Val Ala Trp Tyr Gln Gln Lys Pro Gly Lys Ala Pro Lys Leu Leu Ile
        35                  40                  45

Tyr Ser Ala Ser Phe Leu Tyr Ser Gly Val Pro Ser Arg Phe Ser Gly
    50                  55                  60

Ser Arg Ser Gly Thr Asp Phe Thr Leu Thr Ile Ser Ser Leu Gln Pro
65                  70                  75                  80

Glu Asp Phe Ala Thr Tyr Tyr Cys Gln Gln His Tyr Thr Thr Pro Pro
                85                  90                  95

Thr Phe Gly Gln Gly Thr Lys Val Glu Ile Lys
            100                 105

<210> 7
<211> 120
<212> PRT
<213> Artificial sequence

<220>
<221> MISC_FEATURE
<222> (1)..(120)

<223> Amino acid sequence of the heavy chain variable region of Hmut2 mutant

<400> 7

```
Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Asn Ile Lys Asp Thr
            20                  25                  30

Tyr Ile His Trp Val Arg Gln Ala Pro Gly Lys Gly Leu Glu Trp Val
        35                  40                  45

Ala Arg Ile Tyr Pro Thr Asn Gly Tyr Thr Arg Tyr Ala Asp Ser Val
    50                  55                  60

Lys Gly Arg Phe Thr Ile Ser Ala Asp Thr Ser Lys Asn Thr Ala Tyr
65                  70                  75                  80

Leu Gln Met Asn Ser Leu Arg Ala Glu Asp Thr Ala Val Tyr Tyr Cys
                85                  90                  95

Ser Arg Trp Gly Gly Asp Gly Phe Tyr Ala Met Asp Tyr Trp Gly Gln
            100                 105                 110

Gly Thr Leu Val Thr Val Ser Ser
        115                 120
```

<210> 8
<211> 107
<212> PRT
<213> Artificial sequence

<220>
<221> MISC_FEATURE
<222> (1)..(107)
<223> Amino acid sequence of the light chain variable region of Hmut2 mutant

<400> 8

```
Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Pro Val Asn Thr Ala
            20                  25                  30

Val Ala Trp Tyr Gln Gln Lys Pro Gly Lys Ala Pro Lys Leu Leu Ile
        35                  40                  45

Tyr Ser Ala Ser Phe Leu Tyr Ser Gly Val Pro Ser Arg Phe Ser Gly
    50                  55                  60
```

Ser Arg Ser Gly Thr Asp Phe Thr Leu Thr Ile Ser Ser Leu Gln Pro
65                  70                  75                  80

Glu Asp Phe Ala Thr Tyr Tyr Cys Gln Gln His Tyr Thr Thr Pro Pro
                85                  90                  95

Thr Phe Gly Gln Gly Thr Lys Val Glu Ile Lys
            100                 105

<210>  9
<211>  120
<212>  PRT
<213>  Artificial sequence

<220>
<221>  MISC_FEATURE
<222>  (1)..(120)
<223>  Amino acid sequence of the heavy chain variable region of Hmut3 mutant

<400>  9

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Asn Ile Lys Asp Thr
            20                  25                  30

Tyr Ile His Trp Val Arg Gln Ala Pro Gly Lys Gly Leu Glu Trp Val
        35                  40                  45

Ala Arg Ile Tyr Pro Thr Asn Gly Tyr Thr Arg Tyr Ala Asp Ser Val
    50                  55                  60

Lys Gly Arg Phe Thr Ile Ser Ala Asp Thr Ser Lys Asn Thr Ala Tyr
65                  70                  75                  80

Leu Gln Met Asn Ser Leu Arg Ala Glu Asp Thr Ala Val Tyr Tyr Cys
                85                  90                  95

Ser Arg Trp Gly Gly Asp Gly Phe Tyr Ala Met Asp Tyr Trp Gly Gln
            100                 105                 110

Gly Thr Leu Val Thr Val Ser Ser
        115                 120

<210>  10
<211>  107
<212>  PRT
<213>  Artificial sequence

<220>

<221> MISC_FEATURE
<222> (1)..(107)
<223> Amino acid sequence of the light chain variable region of Hmut3 mutant

<400> 10

```
Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Val Asn Thr Ala
            20                  25                  30

Val Ala Trp Tyr Gln Gln Lys Pro Gly Lys Ala Pro Lys Leu Leu Ile
        35                  40                  45

Tyr Ser Ala Ser Phe Leu Tyr Ser Gly Val Pro Ser Arg Phe Ser Gly
    50                  55                  60

Ser Arg Ser Gly Thr Asp Phe Thr Leu Thr Ile Ser Ser Leu Gln Pro
65                  70                  75                  80

Glu Asp Phe Ala Thr Tyr Tyr Cys Gln Gln His Tyr Tyr Thr Pro Pro
                85                  90                  95

Thr Phe Gly Gln Gly Thr Lys Val Glu Ile Lys
            100                 105
```

<210> 11
<211> 120
<212> PRT
<213> Artificial sequence

<220>
<221> MISC_FEATURE
<222> (1)..(120)
<223> Amino acid sequence of the heavy chain variable region of Hmut4 mutant

<400> 11

```
Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Asn Ile Lys Asp Thr
            20                  25                  30

Tyr Ile His Trp Val Arg Gln Ala Pro Gly Lys Gly Leu Glu Trp Val
        35                  40                  45

Ala Arg Ile Tyr Pro Thr Asn Gly Tyr Thr Arg Tyr Ala Asp Ser Val
    50                  55                  60
```

Lys Gly Arg Phe Thr Ile Ser Ala Asp Thr Ser Lys Asn Thr Ala Tyr
65 70 75 80

Leu Gln Met Asn Ser Leu Arg Ala Glu Asp Thr Ala Val Tyr Tyr Cys
85 90 95

Ser Arg Trp Gly Gly Asp Gly Phe Tyr Ala Met Asp Tyr Trp Gly Gln
100 105 110

Gly Thr Leu Val Thr Val Ser Ser
115 120

<210> 12
<211> 107
<212> PRT
<213> Artificial sequence

<220>
<221> MISC_FEATURE
<222> (1)..(107)
<223> Amino acid sequence of the light chain variable region of Hmut4 mutant
<400> 12

Asp Ile Gln Met Thr Gln Ser Pro Ser Ser Leu Ser Ala Ser Val Gly
1 5 10 15

Asp Arg Val Thr Ile Thr Cys Arg Ala Ser Gln Asp Val Asn Thr Ala
20 25 30

Val Ala Trp Tyr Gln Gln Lys Pro Gly Lys Ala Pro Lys Leu Leu Ile
35 40 45

Tyr Ser Ala Ser Phe Leu Tyr Ser Gly Val Pro Ser Arg Phe Ser Gly
50 55 60

Ser Arg Ser Gly Thr Asp Phe Thr Leu Thr Ile Ser Ser Leu Gln Pro
65 70 75 80

Glu Asp Phe Ala Thr Tyr Tyr Cys Gln Gln His Tyr Asn Thr Pro Pro
85 90 95

Thr Phe Gly Gln Gly Thr Lys Val Glu Ile Lys
100 105

<210> 13
<211> 120
<212> PRT
<213> Artificial sequence

<220>
<221> MISC_FEATURE
<222> (1)..(120)

<223> Amino acid sequence of the heavy chain variable region of Hmut5 mutant

<400> 13

Glu Val Gln Leu Val Glu Ser Gly Gly Gly Leu Val Gln Pro Gly Gly
1 5 10 15

Ser Leu Arg Leu Ser Cys Ala Ala Ser Gly Phe Asn Ile Lys Asp Thr
20 25 30

Tyr Ile His Trp Val Arg Gln Ala Pro Gly Lys Gly Leu Glu Trp Val
35 40 45

Ala Arg Ile Tyr Pro Thr Pro Gly Tyr Thr Arg Tyr Ala Asp Ser Val
50 55 60

Lys Gly Arg Phe Thr Ile Ser Ala Asp Thr Ser Lys Asn Thr Ala Tyr
65 70 75 80

Leu Gln Met Asn Ser Leu Arg Ala Glu Asp Thr Ala Val Tyr Tyr Cys
85 90 95

Ser Arg Trp Gly Gly Asp Gly Phe Tyr Ala Met Asp Tyr Trp Gly Gln
100 105 110

Gly Thr Leu Val Thr Val Ser Ser
115 120

<210> 14
<211> 107
<212> PRT
<213> Artificial sequence

<220>
<221> MISC_FEATURE
<222> (1)..(107)
<223> Amino acid sequence of the light chain variable region of Hmut5 mutant

<400> 14

Asp Ile Gln Met Thr Gln Ser Pro Ser Ser Leu Ser Ala Ser Val Gly
1 5 10 15

Asp Arg Val Thr Ile Thr Cys Arg Ala Ser Gln Asp Val Asn Thr Ala
20 25 30

Val Ala Trp Tyr Gln Gln Lys Pro Gly Lys Ala Pro Lys Leu Leu Ile
35 40 45

Tyr Ser Ala Ser Phe Leu Tyr Ser Gly Val Pro Ser Arg Phe Ser Gly
50 55 60

```
Ser Arg Ser Gly Thr Asp Phe Thr Leu Thr Ile Ser Ser Leu Gln Pro
65                  70                  75                  80

Glu Asp Phe Ala Thr Tyr Tyr Cys Gln Gln His Tyr Thr Thr Pro Pro
                85                  90                  95

Thr Phe Gly Gln Gly Thr Lys Val Glu Ile Lys
            100                 105


<210>  15
<211>  120
<212>  PRT
<213>  Artificial sequence


<220>
<221>  MISC_FEATURE
<222>  (1)..(120)
<223>  Amino acid sequence of the heavy chain variable region of Hmut6 mutant

<400>  15

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Asn Ile Lys Asp Thr
            20                  25                  30

Tyr Ile His Trp Val Arg Gln Ala Pro Gly Lys Gly Leu Glu Trp Val
        35                  40                  45

Ala Arg Ile Tyr Pro Thr Lys Gly Tyr Thr Arg Tyr Ala Asp Ser Val
    50                  55                  60

Lys Gly Arg Phe Thr Ile Ser Ala Asp Thr Ser Lys Asn Thr Ala Tyr
65                  70                  75                  80

Leu Gln Met Asn Ser Leu Arg Ala Glu Asp Thr Ala Val Tyr Tyr Cys
                85                  90                  95

Ser Arg Trp Gly Gly Asp Gly Phe Tyr Ala Met Asp Tyr Trp Gly Gln
            100                 105                 110

Gly Thr Leu Val Thr Val Ser Ser
        115                 120


<210>  16
<211>  107
<212>  PRT
<213>  Artificial sequence


<220>
```

<221> MISC_FEATURE
<222> (1)..(107)
<223> Amino acid sequence of the light chain variable region of Hmut6 mutant

<400> 16

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Val Asn Thr Ala
            20                  25                  30

Val Ala Trp Tyr Gln Gln Lys Pro Gly Lys Ala Pro Lys Leu Leu Ile
        35                  40                  45

Tyr Ser Ala Ser Phe Leu Tyr Ser Gly Val Pro Ser Arg Phe Ser Gly
    50                  55                  60

Ser Arg Ser Gly Thr Asp Phe Thr Leu Thr Ile Ser Ser Leu Gln Pro
65                  70                  75                  80

Glu Asp Phe Ala Thr Tyr Tyr Cys Gln Gln His Tyr Thr Thr Pro Pro
                85                  90                  95

Thr Phe Gly Gln Gly Thr Lys Val Glu Ile Lys
            100                 105

<210> 17
<211> 120
<212> PRT
<213> Artificial sequence

<220>
<221> MISC_FEATURE
<222> (1)..(120)
<223> Amino acid sequence of the heavy chain variable region of Hmut7 mutant

<400> 17

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Asn Ile Lys Asp Thr
            20                  25                  30

Tyr Ile His Trp Val Arg Gln Ala Pro Gly Lys Gly Leu Glu Trp Val
        35                  40                  45

Ala Arg Ile Tyr Pro Thr Asn Gly Tyr Thr Lys Tyr Ala Asp Ser Val
    50                  55                  60

Lys Gly Arg Phe Thr Ile Ser Ala Asp Thr Ser Lys Asn Thr Ala Tyr
65 70 75 80

Leu Gln Met Asn Ser Leu Arg Ala Glu Asp Thr Ala Val Tyr Tyr Cys
85 90 95

Ser Arg Trp Gly Gly Asp Gly Phe Tyr Ala Met Asp Tyr Trp Gly Gln
100 105 110

Gly Thr Leu Val Thr Val Ser Ser
115 120

<210> 18
<211> 107
<212> PRT
<213> Artificial sequence

<220>
<221> MISC_FEATURE
<222> (1)..(107)
<223> Amino acid sequence of the light chain variable region of Hmut7 mutant

<400> 18

Asp Ile Gln Met Thr Gln Ser Pro Ser Ser Leu Ser Ala Ser Val Gly
1 5 10 15

Asp Arg Val Thr Ile Thr Cys Arg Ala Ser Gln Asp Val Asn Thr Ala
20 25 30

Val Ala Trp Tyr Gln Gln Lys Pro Gly Lys Ala Pro Lys Leu Leu Ile
35 40 45

Tyr Ser Ala Ser Phe Leu Tyr Ser Gly Val Pro Ser Arg Phe Ser Gly
50 55 60

Ser Arg Ser Gly Thr Asp Phe Thr Leu Thr Ile Ser Ser Leu Gln Pro
65 70 75 80

Glu Asp Phe Ala Thr Tyr Tyr Cys Gln Gln His Tyr Thr Thr Pro Pro
85 90 95

Thr Phe Gly Gln Gly Thr Lys Val Glu Ile Lys
100 105

<210> 19
<211> 120
<212> PRT
<213> Artificial sequence

<220>

<221> MISC_FEATURE
<222> (1)..(120)
<223> Amino acid sequence of the heavy chain variable region of Hmut8 mutant

<400> 19

```
Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Asn Ile Lys Asp Thr
            20                  25                  30

Tyr Ile His Trp Val Arg Gln Ala Pro Gly Lys Gly Leu Glu Trp Val
        35                  40                  45

Ala Arg Ile Tyr Pro Thr Asn Gly Tyr Thr Arg Tyr Ala Asp Ser Val
    50                  55                  60

Lys Gly Arg Phe Thr Ile Ser Ala Asp Thr Ser Lys Asn Thr Ala Tyr
65                  70                  75                  80

Leu Gln Met Asn Ser Leu Arg Ala Glu Asp Thr Ala Val Tyr Tyr Cys
                85                  90                  95

Ser Arg Trp Gly Gly Thr Gly Phe Tyr Ala Met Asp Tyr Trp Gly Gln
            100                 105                 110

Gly Thr Leu Val Thr Val Ser Ser
        115                 120
```

<210> 20
<211> 107
<212> PRT
<213> Artificial sequence

<220>
<221> MISC_FEATURE
<222> (1)..(107)
<223> Amino acid sequence of the light chain variable region of Hmut8 mutant

<400> 20

```
Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Val Asn Thr Ala
            20                  25                  30

Val Ala Trp Tyr Gln Gln Lys Pro Gly Lys Ala Pro Lys Leu Leu Ile
        35                  40                  45

Tyr Ser Ala Ser Phe Leu Tyr Ser Gly Val Pro Ser Arg Phe Ser Gly
    50                  55                  60
```

```
Ser Arg Ser Gly Thr Asp Phe Thr Leu Thr Ile Ser Ser Leu Gln Pro
65                  70                  75                  80

Glu Asp Phe Ala Thr Tyr Tyr Cys Gln Gln His Tyr Thr Thr Pro Pro
                85                  90                  95

Thr Phe Gly Gln Gly Thr Lys Val Glu Ile Lys
            100                 105


<210>  21
<211>  120
<212>  PRT
<213>  Artificial sequence


<220>
<221>  MISC_FEATURE
<222>  (1)..(120)
<223>  Amino acid sequence of the heavy chain variable region of Hmut9 mutant

<400>  21

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Asn Ile Lys Asp Thr
            20                  25                  30

Tyr Ile His Trp Val Arg Gln Ala Pro Gly Lys Gly Leu Glu Trp Val
        35                  40                  45

Ala Arg Ile Tyr Pro Thr Asn Gly Tyr Thr Arg Tyr Ala Asp Ser Val
    50                  55                  60

Lys Gly Arg Phe Thr Ile Ser Ala Asp Thr Ser Lys Asn Thr Ala Tyr
65                  70                  75                  80

Leu Gln Met Asn Ser Leu Arg Ala Glu Asp Thr Ala Val Tyr Tyr Cys
                85                  90                  95

Ser Arg Trp Gly Gly Tyr Gly Phe Tyr Ala Met Asp Tyr Trp Gly Gln
            100                 105                 110

Gly Thr Leu Val Thr Val Ser Ser
        115                 120


<210>  22
<211>  107
<212>  PRT
<213>  Artificial sequence
```

<220>
<221> MISC_FEATURE
<222> (1)..(107)
<223> Amino acid sequence of the light chain variable region of Hmut9 mutant

<400> 22

```
Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Val Asn Thr Ala
            20                  25                  30

Val Ala Trp Tyr Gln Gln Lys Pro Gly Lys Ala Pro Lys Leu Leu Ile
        35                  40                  45

Tyr Ser Ala Ser Phe Leu Tyr Ser Gly Val Pro Ser Arg Phe Ser Gly
    50                  55                  60

Ser Arg Ser Gly Thr Asp Phe Thr Leu Thr Ile Ser Ser Leu Gln Pro
65                  70                  75                  80

Glu Asp Phe Ala Thr Tyr Tyr Cys Gln Gln His Tyr Thr Thr Pro Pro
                85                  90                  95

Thr Phe Gly Gln Gly Thr Lys Val Glu Ile Lys
            100                 105
```

<210> 23
<211> 120
<212> PRT
<213> Artificial sequence

<220>
<221> MISC_FEATURE
<222> (1)..(120)
<223> Amino acid sequence of the heavy chain variable region of Hmut10 mutant

<400> 23

```
Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Asn Ile Lys Asp Thr
            20                  25                  30

Tyr Ile His Trp Val Arg Gln Ala Pro Gly Lys Gly Leu Glu Trp Val
        35                  40                  45

Ala Arg Ile Tyr Pro Thr Asn Gly Tyr Thr Arg Tyr Ala Asp Ser Val
    50                  55                  60
```

Lys Gly Arg Phe Thr Ile Ser Ala Asp Thr Ser Lys Asn Thr Ala Tyr
65 70 75 80

Leu Gln Met Asn Ser Leu Arg Ala Glu Asp Thr Ala Val Tyr Tyr Cys
85 90 95

Ser Arg Trp Gly Gly Lys Gly Phe Tyr Ala Met Asp Tyr Trp Gly Gln
100 105 110

Gly Thr Leu Val Thr Val Ser Ser
115 120

<210> 24
<211> 107
<212> PRT
<213> Artificial sequence

<220>
<221> MISC_FEATURE
<222> (1)..(107)
<223> Amino acid sequence of the light chain variable region of Hmut10 mutant

<400> 24

Asp Ile Gln Met Thr Gln Ser Pro Ser Ser Leu Ser Ala Ser Val Gly
1 5 10 15

Asp Arg Val Thr Ile Thr Cys Arg Ala Ser Gln Asp Val Asn Thr Ala
20 25 30

Val Ala Trp Tyr Gln Gln Lys Pro Gly Lys Ala Pro Lys Leu Leu Ile
35 40 45

Tyr Ser Ala Ser Phe Leu Tyr Ser Gly Val Pro Ser Arg Phe Ser Gly
50 55 60

Ser Arg Ser Gly Thr Asp Phe Thr Leu Thr Ile Ser Ser Leu Gln Pro
65 70 75 80

Glu Asp Phe Ala Thr Tyr Tyr Cys Gln Gln His Tyr Thr Thr Pro Pro
85 90 95

Thr Phe Gly Gln Gly Thr Lys Val Glu Ile Lys
100 105

<210> 25
<211> 121
<212> PRT
<213> Artificial sequence

<220>
<221> MISC_FEATURE
<222> (1)..(121)
<223> Amino acid sequence of the heavy chain variable region of Rmut1 mutant

<400> 25

```
Gln Val Gln Leu Gln Gln Pro Gly Ala Glu Leu Val Lys Pro Gly Ala
1               5                   10                  15

Ser Val Lys Met Ser Cys Lys Ala Ser Gly Tyr Thr Phe Thr Ser Tyr
            20                  25                  30

Asn Met His Trp Val Lys Gln Thr Pro Gly Arg Gly Leu Glu Trp Ile
        35                  40                  45

Gly Ala Ile Tyr Pro Gly Glu Gly Asp Thr Ser Tyr Asn Gln Lys Phe
    50                  55                  60

Lys Gly Lys Ala Thr Leu Thr Ala Asp Lys Ser Ser Ser Thr Ala Tyr
65                  70                  75                  80

Met Gln Leu Ser Ser Leu Thr Ser Glu Asp Ser Ala Val Tyr Tyr Cys
                85                  90                  95

Ala Arg Ser Thr Tyr Tyr Gly Gly Asp Trp Tyr Phe Asn Val Trp Gly
            100                 105                 110

Ala Gly Thr Thr Val Thr Val Ser Ala
        115                 120
```

<210> 26
<211> 106
<212> PRT
<213> Artificial sequence

<220>
<221> MISC_FEATURE
<222> (1)..(106)
<223> Amino acid sequence of the light chain variable region of Rmut1 mutant

<400> 26

```
Gln Ile Val Leu Ser Gln Ser Pro Ala Ile Leu Ser Ala Ser Pro Gly
1               5                   10                  15

Glu Lys Val Thr Met Thr Cys Arg Ala Ser Ser Ser Val Ser Tyr Ile
            20                  25                  30

His Trp Phe Gln Gln Lys Pro Gly Ser Ser Pro Lys Pro Trp Ile Tyr
        35                  40                  45
```

Ala Thr Ser Asn Leu Ala Ser Gly Val Pro Val Arg Phe Ser Gly Ser
50 55 60

Gly Ser Gly Thr Ser Tyr Ser Leu Thr Ile Ser Arg Val Glu Ala Glu
65 70 75 80

Asp Ala Ala Thr Tyr Tyr Cys Gln Gln Trp Thr Ser Asn Pro Pro Thr
85 90 95

Phe Gly Gly Gly Thr Lys Leu Glu Ile Lys
100 105

<210> 27
<211> 121
<212> PRT
<213> Artificial sequence

<220>
<221> MISC_FEATURE
<222> (1)..(121)
<223> Amino acid sequence of the heavy chain variable region of Rmut2 mutant

<400> 27

Gln Val Gln Leu Gln Gln Pro Gly Ala Glu Leu Val Lys Pro Gly Ala
1 5 10 15

Ser Val Lys Met Ser Cys Lys Ala Ser Gly Tyr Thr Phe Thr Ser Tyr
20 25 30

Asn Met His Trp Val Lys Gln Thr Pro Gly Arg Gly Leu Glu Trp Ile
35 40 45

Gly Ala Ile Tyr Pro Gly Arg Gly Asp Thr Ser Tyr Asn Gln Lys Phe
50 55 60

Lys Gly Lys Ala Thr Leu Thr Ala Asp Lys Ser Ser Ser Thr Ala Tyr
65 70 75 80

Met Gln Leu Ser Ser Leu Thr Ser Glu Asp Ser Ala Val Tyr Tyr Cys
85 90 95

Ala Arg Ser Thr Tyr Tyr Gly Gly Asp Trp Tyr Phe Asn Val Trp Gly
100 105 110

Ala Gly Thr Thr Val Thr Val Ser Ala
115 120

<210> 28
<211> 106
<212> PRT

<213>  Artificial sequence

<220>
<221>  MISC_FEATURE
<222>  (1)..(106)
<223>  Amino acid sequence of the light chain variable region of Rmut2 mutant

<400>  28

```
Gln Ile Val Leu Ser Gln Ser Pro Ala Ile Leu Ser Ala Ser Pro Gly
1               5                   10                  15

Glu Lys Val Thr Met Thr Cys Arg Ala Ser Ser Ser Val Ser Tyr Ile
            20                  25                  30

His Trp Phe Gln Gln Lys Pro Gly Ser Ser Pro Lys Pro Trp Ile Tyr
        35                  40                  45

Ala Thr Ser Asn Leu Ala Ser Gly Val Pro Val Arg Phe Ser Gly Ser
    50                  55                  60

Gly Ser Gly Thr Ser Tyr Ser Leu Thr Ile Ser Arg Val Glu Ala Glu
65                  70                  75                  80

Asp Ala Ala Thr Tyr Tyr Cys Gln Gln Trp Thr Ser Asn Pro Pro Thr
                85                  90                  95

Phe Gly Gly Gly Thr Lys Leu Glu Ile Lys
            100                 105
```

<210>  29
<211>  121
<212>  PRT
<213>  Artificial sequence

<220>
<221>  MISC_FEATURE
<222>  (1)..(121)
<223>  Amino acid sequence of the heavy chain variable region of Rmut3 mutant

<400>  29

```
Gln Val Gln Leu Gln Gln Pro Gly Ala Glu Leu Val Lys Pro Gly Ala
1               5                   10                  15

Ser Val Lys Met Ser Cys Lys Ala Ser Gly Tyr Thr Phe Thr Ser Tyr
            20                  25                  30

Asn Met His Trp Val Lys Gln Thr Pro Gly Arg Gly Leu Glu Trp Ile
        35                  40                  45
```

```
Gly Ala Ile Tyr Pro Gly Asn Gly Glu Thr Ser Tyr Asn Gln Lys Phe
    50                  55                  60


Lys Gly Lys Ala Thr Leu Thr Ala Asp Lys Ser Ser Ser Thr Ala Tyr
65                  70                  75                  80


Met Gln Leu Ser Ser Leu Thr Ser Glu Asp Ser Ala Val Tyr Tyr Cys
                85                  90                  95


Ala Arg Ser Thr Tyr Tyr Gly Gly Asp Trp Tyr Phe Asn Val Trp Gly
            100                 105                 110


Ala Gly Thr Thr Val Thr Val Ser Ala
        115                 120


<210>  30
<211>  106
<212>  PRT
<213>  Artificial sequence


<220>
<221>  MISC_FEATURE
<222>  (1)..(106)
<223>  Amino acid sequence of the light chain variable region of Rmut3 mutant


<400>  30

Gln Ile Val Leu Ser Gln Ser Pro Ala Ile Leu Ser Ala Ser Pro Gly
1               5                   10                  15


Glu Lys Val Thr Met Thr Cys Arg Ala Ser Ser Ser Val Ser Tyr Ile
            20                  25                  30


His Trp Phe Gln Gln Lys Pro Gly Ser Ser Pro Lys Pro Trp Ile Tyr
        35                  40                  45


Ala Thr Ser Asn Leu Ala Ser Gly Val Pro Val Arg Phe Ser Gly Ser
    50                  55                  60


Gly Ser Gly Thr Ser Tyr Ser Leu Thr Ile Ser Arg Val Glu Ala Glu
65                  70                  75                  80


Asp Ala Ala Thr Tyr Tyr Cys Gln Gln Trp Thr Ser Asn Pro Pro Thr
                85                  90                  95


Phe Gly Gly Gly Thr Lys Leu Glu Ile Lys
            100                 105


<210>  31
<211>  121
<212>  PRT
```

<213> Artificial sequence

<220>
<221> MISC_FEATURE
<222> (1)..(121)
<223> Amino acid sequence of the heavy chain variable region of Rmut4 mutant

<400> 31

```
Gln Val Gln Leu Gln Gln Pro Gly Ala Glu Leu Val Lys Pro Gly Ala
1               5                   10                  15

Ser Val Lys Met Ser Cys Lys Ala Ser Gly Tyr Thr Phe Thr Ser Tyr
            20                  25                  30

Asn Met His Trp Val Lys Gln Thr Pro Gly Arg Gly Leu Glu Trp Ile
        35                  40                  45

Gly Ala Ile Tyr Pro Gly Asn Gly Asp Thr Ser Tyr Asn Gln Lys Phe
    50                  55                  60

Lys Gly Lys Ala Thr Leu Thr Ala Asp Lys Ser Ser Ser Thr Ala Tyr
65                  70                  75                  80

Met Gln Leu Ser Ser Leu Thr Ser Glu Asp Ser Ala Val Tyr Tyr Cys
                85                  90                  95

Ala Arg Ser Thr Tyr Arg Gly Gly Asp Trp Tyr Phe Asn Val Trp Gly
            100                 105                 110

Ala Gly Thr Thr Val Thr Val Ser Ala
        115                 120
```

<210> 32
<211> 106
<212> PRT
<213> Artificial sequence

<220>
<221> MISC_FEATURE
<222> (1)..(106)
<223> Amino acid sequence of the light chain variable region of Rmut4 mutant

<400> 32

```
Gln Ile Val Leu Ser Gln Ser Pro Ala Ile Leu Ser Ala Ser Pro Gly
1               5                   10                  15

Glu Lys Val Thr Met Thr Cys Arg Ala Ser Ser Ser Val Ser Tyr Ile
            20                  25                  30
```

```
His Trp Phe Gln Gln Lys Pro Gly Ser Ser Pro Lys Pro Trp Ile Tyr
        35                  40                  45

Ala Thr Ser Asn Leu Ala Ser Gly Val Pro Val Arg Phe Ser Gly Ser
    50                  55                  60

Gly Ser Gly Thr Ser Tyr Ser Leu Thr Ile Ser Arg Val Glu Ala Glu
65                  70                  75                  80

Asp Ala Ala Thr Tyr Tyr Cys Gln Gln Trp Thr Ser Asn Pro Pro Thr
                85                  90                  95

Phe Gly Gly Gly Thr Lys Leu Glu Ile Lys
            100                 105


<210>  33
<211>  121
<212>  PRT
<213>  Artificial sequence


<220>
<221>  MISC_FEATURE
<222>  (1)..(121)
<223>  Amino acid sequence of the heavy chain variable region of Rmut5 mutant


<400>  33

Gln Val Gln Leu Gln Gln Pro Gly Ala Glu Leu Val Lys Pro Gly Ala
1               5                   10                  15

Ser Val Lys Met Ser Cys Lys Ala Ser Gly Tyr Thr Phe Thr Ser Tyr
            20                  25                  30

Asn Met His Trp Val Lys Gln Thr Pro Gly Arg Gly Leu Glu Trp Ile
        35                  40                  45

Gly Ala Ile Tyr Pro Gly Asn Gly Asp Thr Ser Tyr Asn Gln Lys Phe
    50                  55                  60

Lys Gly Lys Ala Thr Leu Thr Ala Asp Lys Ser Ser Ser Thr Ala Tyr
65                  70                  75                  80

Met Gln Leu Ser Ser Leu Thr Ser Glu Asp Ser Ala Val Tyr Tyr Cys
                85                  90                  95

Ala Arg Ser Thr Tyr Ser Gly Gly Asp Trp Tyr Phe Asn Val Trp Gly
            100                 105                 110

Ala Gly Thr Thr Val Thr Val Ser Ala
        115                 120
```

<210> 34
<211> 106
<212> PRT
<213> Artificial sequence

<220>
<221> MISC_FEATURE
<222> (1)..(106)
<223> Amino acid sequence of the light chain variable region of Rmut5 mutant

<400> 34

```
Gln Ile Val Leu Ser Gln Ser Pro Ala Ile Leu Ser Ala Ser Pro Gly
1               5                   10                  15

Glu Lys Val Thr Met Thr Cys Arg Ala Ser Ser Ser Val Ser Tyr Ile
            20                  25                  30

His Trp Phe Gln Gln Lys Pro Gly Ser Ser Pro Lys Pro Trp Ile Tyr
        35                  40                  45

Ala Thr Ser Asn Leu Ala Ser Gly Val Pro Val Arg Phe Ser Gly Ser
    50                  55                  60

Gly Ser Gly Thr Ser Tyr Ser Leu Thr Ile Ser Arg Val Glu Ala Glu
65                  70                  75                  80

Asp Ala Ala Thr Tyr Tyr Cys Gln Gln Trp Thr Ser Asn Pro Pro Thr
                85                  90                  95

Phe Gly Gly Gly Thr Lys Leu Glu Ile Lys
            100                 105
```

<210> 35
<211> 121
<212> PRT
<213> Artificial sequence

<220>
<221> MISC_FEATURE
<222> (1)..(121)
<223> Amino acid sequence of the heavy chain variable region of Rmut6 mutant

<400> 35

```
Gln Val Gln Leu Gln Gln Pro Gly Ala Glu Leu Val Lys Pro Gly Ala
1               5                   10                  15

Ser Val Lys Met Ser Cys Lys Ala Ser Gly Tyr Thr Phe Thr Ser Tyr
            20                  25                  30
```

Asn Met His Trp Val Lys Gln Thr Pro Gly Arg Gly Leu Glu Trp Ile
35 40 45

Gly Ala Ile Tyr Pro Gly Asn Gly Asp Thr Ser Tyr Asn Gln Lys Phe
50 55 60

Lys Gly Lys Ala Thr Leu Thr Ala Asp Lys Ser Ser Ser Thr Ala Tyr
65 70 75 80

Met Gln Leu Ser Ser Leu Thr Ser Glu Asp Ser Ala Val Tyr Tyr Cys
85 90 95

Ala Arg Ser Thr Tyr Thr Gly Gly Asp Trp Tyr Phe Asn Val Trp Gly
100 105 110

Ala Gly Thr Thr Val Thr Val Ser Ala
115 120

<210> 36
<211> 106
<212> PRT
<213> Artificial sequence

<220>
<221> MISC_FEATURE
<222> (1)..(106)
<223> Amino acid sequence of the light chain variable region of Rmut6 mutant

<400> 36

Gln Ile Val Leu Ser Gln Ser Pro Ala Ile Leu Ser Ala Ser Pro Gly
1 5 10 15

Glu Lys Val Thr Met Thr Cys Arg Ala Ser Ser Ser Val Ser Tyr Ile
20 25 30

His Trp Phe Gln Gln Lys Pro Gly Ser Ser Pro Lys Pro Trp Ile Tyr
35 40 45

Ala Thr Ser Asn Leu Ala Ser Gly Val Pro Val Arg Phe Ser Gly Ser
50 55 60

Gly Ser Gly Thr Ser Tyr Ser Leu Thr Ile Ser Arg Val Glu Ala Glu
65 70 75 80

Asp Ala Ala Thr Tyr Tyr Cys Gln Gln Trp Thr Ser Asn Pro Pro Thr
85 90 95

Phe Gly Gly Gly Thr Lys Leu Glu Ile Lys
100 105

<210> 37
<211> 121
<212> PRT
<213> Artificial sequence

<220>
<221> MISC_FEATURE
<222> (1)..(121)
<223> Amino acid sequence of the heavy chain variable region of Rmut7 mutant

<400> 37

```
Gln Val Gln Leu Gln Gln Pro Gly Ala Glu Leu Val Lys Pro Gly Ala
1               5                   10                  15

Ser Val Lys Met Ser Cys Lys Ala Ser Gly Tyr Thr Phe Thr Ser Tyr
            20                  25                  30

Asn Met His Trp Val Lys Gln Thr Pro Gly Arg Gly Leu Glu Trp Ile
        35                  40                  45

Gly Ala Ile Tyr Pro Gly Asn Gly Asp Thr Ser Tyr Asn Gln Lys Phe
    50                  55                  60

Lys Gly Lys Ala Thr Leu Thr Ala Asp Lys Ser Ser Ser Thr Ala Tyr
65                  70                  75                  80

Met Gln Leu Ser Ser Leu Thr Ser Glu Asp Ser Ala Val Tyr Tyr Cys
                85                  90                  95

Ala Arg Ser Thr Tyr Lys Gly Gly Asp Trp Tyr Phe Asn Val Trp Gly
            100                 105                 110

Ala Gly Thr Thr Val Thr Val Ser Ala
        115                 120
```

<210> 38
<211> 106
<212> PRT
<213> Artificial sequence

<220>
<221> MISC_FEATURE
<222> (1)..(106)
<223> Amino acid sequence of the light chain variable region of Rmut7 mutant

<400> 38

```
Gln Ile Val Leu Ser Gln Ser Pro Ala Ile Leu Ser Ala Ser Pro Gly
1               5                   10                  15
```

```
Glu Lys Val Thr Met Thr Cys Arg Ala Ser Ser Ser Val Ser Tyr Ile
            20                  25                  30

His Trp Phe Gln Gln Lys Pro Gly Ser Ser Pro Lys Pro Trp Ile Tyr
        35                  40                  45

Ala Thr Ser Asn Leu Ala Ser Gly Val Pro Val Arg Phe Ser Gly Ser
    50                  55                  60

Gly Ser Gly Thr Ser Tyr Ser Leu Thr Ile Ser Arg Val Glu Ala Glu
65                  70                  75                  80

Asp Ala Ala Thr Tyr Tyr Cys Gln Gln Trp Thr Ser Asn Pro Pro Thr
                85                  90                  95

Phe Gly Gly Gly Thr Lys Leu Glu Ile Lys
            100                 105


<210>  39
<211>  705
<212>  DNA
<213>  Artificial sequence


<220>
<221>  misc_feature
<222>  (1)..(705)
<223>  Nucleotide sequence of the Fc region

<400>  39
gagcccaaat cttctgacaa aactcacaca tccccaccgt ccccagcacc tgaactcctg      60

gggggaccgt cagtcttcct cttcccccca aaacccaagg acaccctcat gatctcccgg     120

acccctgagg tcacatgcgt ggtggtggac gtgagccacg aagaccctga ggtcaagttc     180

aactggtacg tggacggcgt ggaggtgcat aatgccaaga caaagccgcg ggaggagcag     240

tacaacagca cgtaccgtgt ggtcagcgtc ctcaccgtcc tgcaccagga ctggctgaat     300

ggcaaggagt acaagtgcaa ggtctccaac aaagccctcc cagcccccat cgagaaaacc     360

atctccaaag ccaaagggca gccccgagaa ccacaggtgt acaccctgcc cccatcccgg     420

gatgagctga ccaagaacca ggtcagcctg acctgcctgg tcaaaggctt ctatcccagc     480

gacatcgccg tggagtggga gagcaatggg cagccggaga acaactacaa gaccacgcct     540

cccgtgctgg actccgacgg ctccttcttc ctctacagca agctcaccgt ggacaagagc     600

aggtggcagc aggggaacgt cttctcatgc tccgtgatgc atgaggctct gcacaaccac     660

tacacgcaga agagcctctc cctgtctccg ggtaaatgag aattc                     705


<210>  40
<211>  232
<212>  PRT
<213>  Artificial sequence
```

<220>
<221> MISC_FEATURE
<222> (1)..(232)
<223> Amino acid sequence of the Fc region

<400> 40

```
Glu Pro Lys Ser Ser Asp Lys Thr His Thr Ser Pro Pro Ser Pro Ala
1               5                   10                  15

Pro Glu Leu Leu Gly Gly Pro Ser Val Phe Leu Phe Pro Pro Lys Pro
            20                  25                  30

Lys Asp Thr Leu Met Ile Ser Arg Thr Pro Glu Val Thr Cys Val Val
        35                  40                  45

Val Asp Val Ser His Glu Asp Pro Glu Val Lys Phe Asn Trp Tyr Val
    50                  55                  60

Asp Gly Val Glu Val His Asn Ala Lys Thr Lys Pro Arg Glu Glu Gln
65                  70                  75                  80

Tyr Asn Ser Thr Tyr Arg Val Val Ser Val Leu Thr Val Leu His Gln
                85                  90                  95

Asp Trp Leu Asn Gly Lys Glu Tyr Lys Cys Lys Val Ser Asn Lys Ala
            100                 105                 110

Leu Pro Ala Pro Ile Glu Lys Thr Ile Ser Lys Ala Lys Gly Gln Pro
        115                 120                 125

Arg Glu Pro Gln Val Tyr Thr Leu Pro Pro Ser Arg Asp Glu Leu Thr
    130                 135                 140

Lys Asn Gln Val Ser Leu Thr Cys Leu Val Lys Gly Phe Tyr Pro Ser
145                 150                 155                 160

Asp Ile Ala Val Glu Trp Glu Ser Asn Gly Gln Pro Glu Asn Asn Tyr
                165                 170                 175

Lys Thr Thr Pro Pro Val Leu Asp Ser Asp Gly Ser Phe Phe Leu Tyr
            180                 185                 190

Ser Lys Leu Thr Val Asp Lys Ser Arg Trp Gln Gln Gly Asn Val Phe
        195                 200                 205

Ser Cys Ser Val Met His Glu Ala Leu His Asn His Tyr Thr Gln Lys
    210                 215                 220
```

Ser Leu Ser Leu Ser Pro Gly Lys
225 230

<210> 41
<211> 93
<212> DNA
<213> Artificial sequence

<220>
<221> misc_feature
<222> (1)..(93)
<223> Sequence of synthetic signal peptide

<400> 41
aagcttgccg ccaccatggg tgtactgctc acacagagga cgctgctcag tctggtcctt 60

gcactcctgt ttccaagcat ggcgagcatg gca 93

<210> 42
<211> 125
<212> PRT
<213> Artificial sequence

<220>
<221> MISC_FEATURE
<222> (1)..(125)
<223> CTmut1 amino acid sequence

<400> 42

Met His Val Ala Gln Pro Ala Val Val Leu Ala Ser Ser Arg Gly Ile
1 5 10 15

Ala Ser Phe Val Cys Glu Tyr Ala Ser Pro Gly Lys Tyr Thr Glu Val
20 25 30

Arg Val Thr Val Leu Arg Gln Ala Asp Ser Gln Val Thr Glu Val Cys
35 40 45

Ala Ala Thr Tyr Met Met Gly Asn Glu Leu Thr Phe Leu Asp Asp Ser
50 55 60

Ile Cys Thr Gly Thr Ser Ser Gly Asn Gln Val Asn Leu Thr Ile Gln
65 70 75 80

Gly Leu Arg Ala Met Asp Thr Gly Leu Tyr Ile Cys Lys Val Glu Leu
85 90 95

Met Tyr Pro Pro Pro Tyr Tyr Leu Gly Ile Gly Asn Gly Thr Gln Ile
100 105 110

Tyr Val Ile Asp Pro Glu Pro Cys Pro Asp Ser Asp Gln
115 120 125

<210> 43
<211> 125
<212> PRT
<213> Artificial sequence

<220>
<221> MISC_FEATURE
<222> (1)..(125)
<223> CTmut2 amino acid sequence

<400> 43

```
Met His Val Ala Gln Pro Ala Val Val Leu Ala Ser Ser Arg Gly Ile
1               5                   10                  15

Ala Ser Phe Val Cys Glu Tyr Ala Ser Pro Gly Lys Lys Thr Glu Val
            20                  25                  30

Arg Val Thr Val Leu Arg Gln Ala Asp Ser Gln Val Thr Glu Val Cys
        35                  40                  45

Ala Ala Thr Tyr Met Met Gly Asn Glu Leu Thr Phe Leu Asp Asp Ser
    50                  55                  60

Ile Cys Thr Gly Thr Ser Ser Gly Asn Gln Val Asn Leu Thr Ile Gln
65                  70                  75                  80

Gly Leu Arg Ala Met Asp Thr Gly Leu Tyr Ile Cys Lys Val Glu Leu
                85                  90                  95

Met Tyr Pro Pro Pro Tyr Tyr Leu Gly Ile Gly Asn Gly Thr Gln Ile
            100                 105                 110

Tyr Val Ile Asp Pro Glu Pro Cys Pro Asp Ser Asp Gln
        115                 120                 125
```

<210> 44
<211> 125
<212> PRT
<213> Artificial sequence

<220>
<221> MISC_FEATURE
<222> (1)..(125)
<223> CTmut6 amino acid sequence

<400> 44

```
Met His Val Ala Gln Pro Ala Val Val Leu Ala Ser Ser Arg Gly Ile
1               5                   10                  15

Ala Ser Phe Val Cys Glu Tyr Ala Ser Pro Gly Lys Ala Thr Glu Val
            20                  25                  30
```

Arg Val Thr Val Leu Arg Gln Ala Asp Ser Gln Val Thr Glu Val Cys
35 40 45

Ala Ala Thr Tyr Met Met Gly Asn Glu Leu Thr Phe Tyr Asp Asp Ser
50 55 60

Ile Cys Thr Gly Thr Ser Ser Gly Asn Gln Val Asn Leu Thr Ile Gln
65 70 75 80

Gly Leu Arg Ala Met Asp Thr Gly Leu Tyr Ile Cys Lys Val Glu Leu
85 90 95

Met Tyr Pro Pro Pro Tyr Tyr Leu Gly Ile Gly Asn Gly Thr Gln Ile
100 105 110

Tyr Val Ile Asp Pro Glu Pro Cys Pro Asp Ser Asp Gln
115 120 125

<210> 45
<211> 125
<212> PRT
<213> Artificial sequence

<220>
<221> MISC_FEATURE
<222> (1)..(125)
<223> CTmut10 amino acid sequence

<400> 45

Met His Val Ala Gln Pro Ala Val Val Leu Ala Ser Ser Arg Gly Ile
1 5 10 15

Ala Ser Phe Val Cys Glu Tyr Ala Ser Pro Gly Lys Ala Thr Glu Val
20 25 30

Arg Val Thr Val Leu Arg Gln Ala Asp Ser Gln Val Thr Glu Val Cys
35 40 45

Ala Ala Thr Tyr Met Met Gly Asn Glu Leu Thr Phe Leu Asp Asp Ser
50 55 60

Ile Cys Thr Gly Thr Ser Ser Gly Asn Gln Val Asn Leu Thr Ile Gln
65 70 75 80

Gly Leu Arg Ala Met Asp Thr Gly Leu Tyr Ile Cys Lys Val Glu Leu
85 90 95

Met Tyr Pro Pro Pro Tyr Tyr Ser Gly Ile Gly Asn Gly Thr Gln Ile
100 105 110

```
Tyr Val Ile Asp Pro Glu Pro Cys Pro Asp Ser Asp Gln
        115                 120                 125


<210>  46
<211>  125
<212>  PRT
<213>  Artificial sequence


<220>
<221>  MISC_FEATURE
<222>  (1)..(125)
<223>  CTmut3 amino acid sequence

<400>  46

Met His Val Ala Gln Pro Ala Val Val Leu Ala Ser Ser Arg Gly Ile
1               5                   10                  15


Ala Ser Phe Val Cys Glu Tyr Ala Ser Pro Gly Lys Ala Thr Glu Val
            20                  25                  30


Arg Val Thr Val Leu Arg Gln Ala Asp Ser Gln Val Thr Glu Val Cys
        35                  40                  45


Ala Ala Lys Tyr Met Met Gly Asn Glu Leu Thr Phe Leu Asp Asp Ser
    50                  55                  60


Ile Cys Thr Gly Thr Ser Ser Gly Asn Gln Val Asn Leu Thr Ile Gln
65                  70                  75                  80


Gly Leu Arg Ala Met Asp Thr Gly Leu Tyr Ile Cys Lys Val Glu Leu
                85                  90                  95


Met Tyr Pro Pro Pro Tyr Tyr Leu Gly Ile Gly Asn Gly Thr Gln Ile
            100                 105                 110


Tyr Val Ile Asp Pro Glu Pro Cys Pro Asp Ser Asp Gln
        115                 120                 125


<210>  47
<211>  125
<212>  PRT
<213>  Artificial sequence


<220>
<221>  MISC_FEATURE
<222>  (1)..(125)
<223>  CTmut4 amino acid sequence

<400>  47

Met His Val Ala Gln Pro Ala Val Val Leu Ala Ser Ser Arg Gly Ile
```

```
1               5                   10                  15

Ala Ser Phe Val Cys Glu Tyr Ala Ser Pro Gly Lys Ala Thr Glu Val
            20                  25                  30

Arg Val Thr Val Leu Arg Gln Ala Asp Ser Gln Val Thr Glu Val Cys
        35                  40                  45

Ala Ala Thr Tyr Glu Met Gly Asn Glu Leu Thr Phe Leu Asp Asp Ser
    50                  55                  60

Ile Cys Thr Gly Thr Ser Ser Gly Asn Gln Val Asn Leu Thr Ile Gln
65                  70                  75                  80

Gly Leu Arg Ala Met Asp Thr Gly Leu Tyr Ile Cys Lys Val Glu Leu
                85                  90                  95

Met Tyr Pro Pro Pro Tyr Tyr Leu Gly Ile Gly Asn Gly Thr Gln Ile
            100                 105                 110

Tyr Val Ile Asp Pro Glu Pro Cys Pro Asp Ser Asp Gln
        115                 120                 125
```

<210> 48
<211> 125
<212> PRT
<213> Artificial sequence

<220>
<221> MISC_FEATURE
<222> (1)..(125)
<223> CTmut5 amino acid sequence

<400> 48

```
Met His Val Ala Gln Pro Ala Val Val Leu Ala Ser Ser Arg Gly Ile
1               5                   10                  15

Ala Ser Phe Val Cys Glu Tyr Ala Ser Pro Gly Lys Ala Thr Glu Val
            20                  25                  30

Arg Val Thr Val Leu Arg Gln Ala Asp Ser Gln Val Thr Glu Val Cys
        35                  40                  45

Ala Ala Thr Tyr Met Met Gly Asn Glu Leu Thr Phe Lys Asp Asp Ser
    50                  55                  60

Ile Cys Thr Gly Thr Ser Ser Gly Asn Gln Val Asn Leu Thr Ile Gln
65                  70                  75                  80

Gly Leu Arg Ala Met Asp Thr Gly Leu Tyr Ile Cys Lys Val Glu Leu
```

```
                85                  90                  95

Met Tyr Pro Pro Pro Tyr Tyr Leu Gly Ile Gly Asn Gly Thr Gln Ile
            100                 105                 110

Tyr Val Ile Asp Pro Glu Pro Cys Pro Asp Ser Asp Gln
        115                 120                 125


<210>  49
<211>  125
<212>  PRT
<213>  Artificial sequence


<220>
<221>  MISC_FEATURE
<222>  (1)..(125)
<223>  CTmut7 amino acid sequence

<400>  49

Met His Val Ala Gln Pro Ala Val Val Leu Ala Ser Ser Arg Gly Ile
1               5                   10                  15

Ala Ser Phe Val Cys Glu Tyr Ala Ser Pro Gly Lys Ala Thr Glu Val
            20                  25                  30

Pro Val Thr Val Leu Arg Gln Ala Asp Ser Gln Val Thr Glu Val Cys
        35                  40                  45

Ala Ala Thr Tyr Met Met Gly Asn Glu Leu Thr Phe Leu Asp Asp Ser
    50                  55                  60

Ile Cys Thr Gly Thr Ser Ser Gly Asn Gln Val Asn Leu Thr Ile Gln
65                  70                  75                  80

Gly Leu Arg Ala Met Asp Thr Gly Leu Tyr Ile Cys Lys Val Glu Leu
                85                  90                  95

Met Tyr Pro Pro Pro Tyr Tyr Leu Gly Ile Gly Asn Gly Thr Gln Ile
            100                 105                 110

Tyr Val Ile Asp Pro Glu Pro Cys Pro Asp Ser Asp Gln
        115                 120                 125


<210>  50
<211>  125
<212>  PRT
<213>  Artificial sequence


<220>
<221>  MISC_FEATURE
<222>  (1)..(125)
```

<223> CTmut10 amino acid sequence

<400> 50

```
Met His Val Ala Gln Pro Ala Val Val Leu Ala Ser Ser Arg Gly Ile
1               5                   10                  15

Ala Ser Phe Val Cys Glu Tyr Ala Ser Pro Gly Lys Ala Thr Glu Val
            20                  25                  30

Arg Val Thr Val Leu Arg Gln Ala Asp Ser Gln Val Thr Glu Val Cys
        35                  40                  45

Ala Ala Thr Tyr Met Met Gly Asn Glu Leu Thr Phe Leu Asp Asp Ser
    50                  55                  60

Ile Cys Thr Gly Thr Ser Ser Gly Asn Gln Val Asn Leu Thr Ile Gln
65                  70                  75                  80

Gly Leu Arg Ala Met Asp Thr Gly Leu Tyr Ile Cys Lys Val Glu Leu
                85                  90                  95

Met Tyr Pro Pro Pro Tyr Tyr Asn Gly Ile Gly Asn Gly Thr Gln Ile
            100                 105                 110

Tyr Val Ile Asp Pro Glu Pro Cys Pro Asp Ser Asp Gln
        115                 120                 125
```

**Description**

FIELD OF THE INVENTION

The present invention relates to the field of biotechnology, and in particular to a method of acquiring antibodies or proteins with improved affinity by computer-aided design (CAD).

BACKGROUND OF THE INVENTION

Since the 1980s of the last century, the increased quantity of structure-resolved proteins year by year and the development of user-friendly structure analyzing software enable us to more deeply understand the atomic basis of molecular inter-recognition. Previously, there were some successful examples in the study of the structure-based modification of enzyme specificity, which indicate that modification of protein function may be realized in the near future. So far, researchers have successfully modified enzyme activities in some study models by computer-aided design to improve the antibody affinity, even to create non-naturally occurring catalytic activities by the modification. The improvement of antibody affinity has an important significance for improvement of detection sensitivity, extension of dissociation time, reduction of drug dose and enhancement of drug effect.

At present, the methods of improving antibody affinity mainly employ original parent monoclonal antibodies as modification templates to construct their mutant antibody libraries (such as Ribosome Display, Yeast Two-Hybrid System, Phage Display Antibody Library) for screening and finally acquiring the monoclonal antibodies with higher affinity. However, these technologies have great limitations: it is difficult to construct a mutant library that could cover all sites and mutate to any amino acid; it is time/labor consumptive to construct and screen the antibody libraries; and it is impractical to screen the antibody libraries when target proteins are hardly expressed or unstably combined with their antibodies under in vitro screening circumstances.

In comparison with the previous antibody library technologies, computer-aided design can screen an antibody library through virtual mutation and thus greatly reduce the experimental time; and can perform virtual mutation at a single site or at combination sites among all binding sites of the antibody. Usually, even only one predicted mutated amino acid could significantly improve antibody affinity. However, there are still some problems such as low accuracy and large amount of calculation in the existing computer-aided design methods. For example, in the experiments of protein modification and simulation, the bioinformatics scientists always tried to modify proteins by mutating all amino acids on the protein-ligand contact surface into other amino acids except proline. Because there are large amount of amino acids on the contact surface between proteins, mutating all the amino acids without selection will require considerable calculation and due to the operating speed limitation of the computer, it will take a great number of approximate values to simplify the calculation, which finally not only waste tremendous calculation time, but not necessarily produce a high prediction accuracy. It is necessary and significant to develop a method of acquiring mutant sites with high affinity quickly and accurately, without extending but reducing calculation time.

SUMMARY OF THE INVENTION

The object of the present invention is to provide a method of acquiring antibodies with high affinity by computer-aided design. The method combines antibody evolution laws with computer simulation techniques to increase true positive sites in the computer simulation and significantly enhance the accuracy of prediction of protein affinity.

The inventors summarized the maturation process of the antibody affinity firstly and established a computer-aided design method based on the evolution of the antibody affinity to enhance the antibody affinity quickly and effectively (with accuracy over 57%). In order to verify the commonality of said method, the method was further used in the experiments for improving the affinity of fusion protein receptor and the similar accuracy were obtained. In principles, the method according to the present invention can be widely used to improve the interactions between protein complexes to facilitate the development of the proteins with biological and medical significance. Meanwhile, the combination of antibody evolution laws and computer simulation techniques proposes a new concept for the future computer-aided design.

According to the present invention, the method of improving antibodies affinity by computer-aided design comprises the following steps:

A method of acquiring antibodies or proteins with high affinity by computer-aided design, comprising the steps of:

1) based on a known structure of a cocrystal of a complex of an antibody or a protein molecule, determining candidate sites of virtual mutation of the antibody or the protein molecule;

2) simulating amino acid mutations in candidate sites of virtual mutation in turn by computer so as to acquire preliminary optimized molecular structures;

3) searching out conformations of the preliminary optimized molecular structures by computer, so as to acquire simulated structures of the antibody or the protein molecule after virtual mutation;

4) analyzing total energies and root mean square deviations of the optimized structures of the antibody or the protein molecular, and selecting mutant conformations with minimized energy and less root mean square deviations to analyze binding energies binding to the protein molecule and to acquire simulative structures; and

5) based on the simulative structures, constructing and predicting mutants of the antibody or the protein with improved affinity, and validating the improved affinity by experiments so as to acquire an antibody mutant or a protein mutant with high affinity.

Wherein, in the step 1), based on the known characteristic changes on the structure of the cocrystal during affinity maturation of the antibody or protein, determining the mutation sites; and selecting the amino acids that are biased distributed on the surface and contact surface of the protein complex as candidate mutated amino acids. The selected mutation sites are located at the periphery of the contact surface between an antibody or protein molecule and an antigen or binding protein, and do not interact with the antigen or binding protein.

Wherein, in the step 2), said virtual mutation sites are mutated into an amino acid selected from the group consisting of Glu, Arg, Asn, Ser, Thr, Tyr, Lys, Asp, Pro and/or Ala.

Wherein, the step 4) comprises the steps of:

a) sorting the preliminary optimized antibody or protein molecule of step 3) according to the overall energy;

b) based on the cocrystal structure of complex of the antibody or protein molecule complex, determining key amino acids involved in binding on the target molecule;

c) mutating the key amino acids involved in binding, simulating the optimized structures and crystal structures and analyzing the root mean square deviations, selecting the mutant structures with minimized total energies and less root mean square deviations to calculate, analyze and sort their binding energies;

d) based on the sorting results of step c), acquiring the simulative structures with high affinity of the antibody or the protein molecule.

**Selection of mutation sites**

In the present invention, the selection of mutation sites mainly comprises based on the known characteristic changes on the structure of the crystal during affinity maturation of the antibody, selecting the amino acids that are biased distributed on the surface and contact surface of the protein complex as candidate mutated amino acids.

Strategy of selecting mutation should first meet the following requirements:

1) The mutation sites are preferably located in the CDR region to avoid the possible immunogenicity as much as possible;

ii) The mutation sites should not be too many and the affinity can be significantly and cooperatively improved at the limited sites, without excessively altering the contact surface of the antibody;

iii) The final method should have high efficiency and high accuracy, and can quickly acquire an antibody with improved affinity by the limited mutation.

The mutation sites selected according to the present invention have the following two features: i) to ensure that a single site mutation has the possible enlarged positions; ii) to ensure that a combined mutation has the best concertedness, thereby greatly improving the affinity of an amino acid antibody.

Clark L.A. *et al.* has carried out mathematical and statistical analysis on the antigen-antibody cocrystals in the PDB database and has acquired the bias of the amino acids widely distributed on the contact surface of the antibody by information searching technology (see Fig.2, Clark L A, Ganesan S, Papp S, et al. Trends in antibody sequence changes during the somatic hypermutation process.[J]. J Immunol. 2006, 177(1): 333-340; Lo C L, Chothia C, Janin J.

The atomic structure of protein-protein recognition sites.[J]. J Mol Biol. 1999, 285(5): 2177-2198). According to the above-mentioned bias of the distribution of the amino acids, the amino acids that are present on the contact surface and surface of the antibody with higher probability are selected as candidate mutated amino acid. Base on the existing accuracy of prediction, by selecting purposively, it is possible to exclude the predicted false-positive amino acids that are rarely present on the contact surface of the antibody and thus improve the accuracy of prediction.

According to the research of Reichmann *et al.,* the contact surface of proteins is distributed in clusters. The amino acids mutations within the cluster always do not have a great synergistic effect. However, the amino acids mutations happened between different clusters could create a maximal synergistic effect between the amino acids. Meanwhile, during the affinity maturation of the antibody, the central area of the contact surface usually makes more contribution to the affinity and evolves more completely; while the periphery of the contact surface has poor antibody affinity and always evolves incompletely due to the limitation of in vivo affinity maturation and the endocytosis of the antigens. Therefore, according to the present invention, the amino acid sites at the periphery of the contact surface between antigen and antibody are selected as mutation sites and it is preferable to select those amino acid sites that do not interact with the antigen.

Consequently, the antibody mutation sites selected according to the present invention have the following features: (1) the selected mutation sites are located at the periphery of the contact surface and should better not interact with the antigen materials; (2) the selected mutation sites are mutated into an amino acid selected from the group consisting of Glu, Arg, Asn, Ser, Thr, Tyr, Lys, Asp, Pro and Ala.

**Mutation method by computer simulation**

PDB files obtained from the PDB database (PDB; Berman, Westbrook et al. (2000), Nucleic Acids Res. 28, 235-242; http://www.pdb.org/) are imported into InsightII (Accelrys). Using consistent valence force field (CVFF) (Pnina D O. Structure and energetics of ligand binding to proteins: Escherichia coli dihydrofolate reductase-trimethoprim, a drug-receptor system[J]. Proteins: Structure, Function, and Genetics. 1988, 4(1): 31-47*),* hydrogen atoms are added by Biopolymer module (a module in InsightII software package). 5000 steps of energy minimization are performed on the hydrogen bond while keeping all heavy atoms of a protein fixed to their positions (with step size of 1fs). The optimized structure with minimized energy are obtained, and the distance of 6Å from the antigen is set as contact surface and water molecules are added at the distance of 25Å around the contact surface. The selected amino acid sites were subjected to amino acid mutation, and the amino acid molecules at a distance of 6Å from the mutation sites were subjected to auto_rotamer to select an optimal space initiation sites (Dunbrack R L. Rotamer Libraries in the 21st Century [J]. Current Opinion in Structural Biology. 2002, 12(4): 431-440. Ponder J W, Richards F M. Tertiary templates for proteins : Use of packing criteria in the enumeration of allowed sequences for different structural classes[J]. Journal of Molecular Biology. 1987, 193(4): 775-791*).* The water molecules at the periphery of the protein complex and the antibody molecules out of the contact surface of the protein complex are subjected to constraint and simulated annealing to find the most likely contact mode.

The Quartic VDW (van der waals) with coulombic interactions off method is firstly used to select the possible binding conformations, wherein the constant of the van der Waals forces and hydrogen bonds in the process is reduced to 0.5 and a 6000-step search is taken for each time, and finally 60 confirmations are obtained. Then, the obtained 60 preliminary optimized conformations are respectively subjected to a more sophisticated search by cell_mutipole method (Ding H Q, Karasawa N, Goddard I I. Atomic level simulations on a million particles: The cell multipole method for Coulomb and London nonbond interactions[J]. J. Chem. Phys. 1992, 97(6): 4309-4315*).*

Herein, the constant of the van der Waals and Coulomb force option is set as 0.5, and 50 stages are divided from temperature of 500K to 280K, with 100fs for each stage, and the final obtained structures are further subjected to a 6000-step energy minimization (Senderowitz H, Guarnieri F, Still W C. A Smart Monte Carlo Technique for Free Energy Simulations of Multiconformational Molecules. Direct Calculations of the Conformational Populations of Organic Molecules [J]. J. Am. Chem. Soc. 1995, 117(31): 8211-8219*).* The binding energies, total energies and root mean square deviations (RMSD) of the obtained structures are scored and the conformations with minimized total energy and less RMSD are selected out.

The selected complexes are imported into charmm V34b1 (Bernard, R. B. and E. B. Robert, et al. (1983). "CHARMM: A program for macromolecular energy, minimization, and dynamics calculations." J Comput Chem. 4(2): 187-217). Hydrogen atoms are added to the heavy atoms of the PDB structure by HBUILD order using charmm force field (Becker, O. M. and M. Karplus (2005). Guide to Biomolecular Simulations (Focus on Structural Biology) for charmm , Springer*).* Energy minimization of the entire system is carried out with Generalized Born with a simple Switching (GBSW) *(*Im, W., Lee, M. S. & Brooks, C. L. Generalized born model with a simple smoothing function. J. Comput. Chem. 24, 1691-1702 (2003*)* implicit water model (Im, W., Lee, M. S. & Brooks, C. L. Generalized born model with a simple smoothing function. J. Comput. Chem. 24, 1691-1702 (2003*)).* The relative binding energy of the balanced complexes are evaluated by the method of MM-PBSA (Kuhn, B., Gerber, P., Schulz-Gasch, T. & Stahl, M. Validation and use of the MM-PBSA approach for drug discovery. J. Med. Chem. 48, 4040-4048 (2005*).* Alonso, H., Bliznyuk, A. A. & Gready, J. E. Combining docking and molecular dynamic simulations in drug design. Med. Res. Rev. 26, 531-568 (2006*)).*

The binding free energy is evaluated with the following formula:

$$\Delta Gbind = <Emm> + \Delta Gsolv - T\Delta S$$

(Fogolari, F. and A. Brigo, et al. (2003). "protocol for MM/PBSA moleculardynamics simulations of proteins. " Biophys J 85(1): 159-66)

Wherein, Emm is the molecular mechanics energy calculated by CVFF force field; ΔGsolv is solvation free energy; -TΔS is entropy of the solute.

$$<Emm> = <\Delta EvdW> + <\Delta Eelec> + <\Delta Eint>$$

Wherein, the molecular mechanics energy consists of intramolecular energy, van der Waals force and electrostatic interaction. The structure of an antibody does not change when it binds to an antigen or not. Therefore, the internal energy of the molecular mechanics energy has no contribution to the binding free energy.

$$\Delta Gsolv = \Delta GPB + \Delta Gnp$$

ΔGPB is electrostatic solvation energy; ΔGnp is non-polar solvation energy.

Because the mutations only happen at sites of the original antibody and cause minor change, the changes of -TΔS is negligible. Kollman *et al.* carried out dynamics simulations and binding energy analysis of the antibodies with mature affinity and their germline antibodies, and found that ΔGnp and -TΔS changed very little during affinity maturation process and had little effect on the binding energy (Chong L T, Duan Y, Wang L, et al. Molecular dynamics and free-energy calculations applied to affinity maturation in antibody 48G7.[J]. Proc Natl Acad Sci U S A. 1999, 96(25): 14330-14335*).* ΔGPB usually plays a negative role in the binding of proteins, however, the compensation of the protein electrostatic interactions makes a relative stable binding between proteins (Novotny J, Sharp K. Electrostatic fields in antibodies and antibody/ antigen complexes.[J]. Prog Biophys Mol Biol. 1992, 58(3): 203-224. Novotny J, Bruccoleri R E, Davis M, et al. Empirical free energy calculations: a blind test and further improvements to the method.[J]. JMol Biol. 1997, 268(2): 401-411*).* Therefore, we simplify the formula for evaluating the binding energy herein, and only calculate the contribution of the molecular mechanics to the binding energy.

**Conformation search of the mutated structures by computer simulation methods**

First, the method of Quartic VDW (van der waals) with coulombic interactions off is used to optimize the mutated structures and acquire a certain number of preliminary optimized structures.

Because of the large number and high freedom degree of protein molecules, conformation search of protein molecules is still a bottleneck in structure simulation. In preliminary conformation search, simple rigid sphere model is used to evaluate van der waals in the present invention. And the influence of the Coulomb force between molecules is not calculated. Thus, the energy interface becomes smoother and it is relatively easier to pick out the minimized values of local energy. The method of Quartic VDW (van der waals) with coulombic interactions off is usually used to perform preliminary conformation space search. Then the acquired preliminary structures are subjected to a more sophisticated conformation search by cell_mutipole method to acquire the antibodies or protein molecules with optimized energy.

For biological macromolecules, it will take a lot of time to simulate by the method of infinity cutoff directly. It is infeasible even by the fastest computer today. Cell_mutipole is a quick and high effective method, which is specially developed for macromolecular simulation. Cell_mutipole has a calculation scale linearly related to the moleculus of the computing architecture and modest memory demand (Ding, H. Q. and N. Karasawa, et al. (1992). "Atomic level simulations on a million particles: The cell multipole method for Coulomb and London nonbond interactions." J. Chem. Phys. 97(6): 4309-4315*).*

**Comprehensive evaluation of the optimized structures**

The optimized structures are comprehensively evaluated by the indexes of energy scores and root mean square deviation (RMSD), acquiring the predicted antibody mutation sites with improved affinity, which comprises the detailed steps of: scoring the above antibodies or protein molecules with optimized energy according to the total energy from high to low; determining key amino acids involved in binding on the target molecules, according to the crystal structure of the protein complexes; simulating mutations of the key amino acids involved in binding and analyzing the RMSD (heavy atoms) of the crystal structures; selecting the mutant structures with minimized total energy and relative less RMSD to calculate and analyze binding energy; and finally acquiring simulative structures with an improved affinity of the antibody or the protein molecule.

The predicted mutants with an improved affinity of the antibody or the protein molecule are constructed and expressed, and respectively verified by tests relevant to affinity improvement to acquire an mutant with improved affinity of the antibody or the protein.

The present invention develops a method of improving antibody or protein affinity by combining antibody affinity maturation laws with traditional computer simulation techniques. The method according to the present invention significantly improves the accuracy of prediction of protein affinity by computer simulation, and greatly reduces calculation workload and the laboratory costs for improving antibody affinity, which makes the modification of protein affinity become simple and effective.

DESCRIPTION OF DRAWINGS

Fig.1 shows an experimental flow chart of the method according to the present invention.

Fig.2 shows the analysis of the biased distribution of amino acids.

Fig.3 shows the mutation sites capable of improving Trastuzumab affinity verified by experiments; as shown in Fig. 3, Asn, at site 55 of the heavy chain; Asp, at site 102 of the heavy chain; Asp, at site of 28 of the light chain; and Thr, at site 93 of the light chain.

Fig.4 shows the nucleotide sequences and amino acid sequences of the heavy chain variable region (VH) and the light chain variable region (VL) of Trastuzumab.

Fig. 5 shows the mutation sites capable of improving Rituximab affinity verified by experiments; as shown in Fig.5: H57Asp and H102Tyr.

Fig.6 shows the nucleotide sequences and amino acid sequences of the heavy chain variable region (VH) and the light chain variable region (VL) of Rituximab.

Fig.7 shows the sensorgram of Rituximab and Rituximab mutants detected by biacore at the same concentration of the samples.

Fig.8 shows the nucleotide sequence and amino acid sequence of CTLA-4 extracellular domain.

Fig.9 shows the sensorgram of Abatacept and CTLA-4/Ig mutants detect by biacore at the same concentration of the samples.

DETAILED DESCRIPTION OF THE INVENTION

The experimental methods of improving the affinity of mature antibodies (Trastuzumab and Rituximab) and fusion protein receptor (CTLA4-Ig) are described in the following embodiments. The features and advantages of the present invention can be further understood by these embodiments.

## Experiment of improving the antibody affinity of Trastuzumab

Trastuzumab (Herceptin) is a humanized monoclonal antibody that specially targets HER2, which is developed by Genentech (USA). It has high affinity for HER2 receptor and is used for the treatment of HER2/neu overexpressing metastatic breast cancer.

Trastuzumab with same epitope and super high affinity is acquired by stimulating the process of affinity improvement in vitro by computer in the present invention, which overcomes limitations of the affinity maturation process in vivo. Finally, a new type of Tratuzumab with stronger anti-tumor activity is acquired and verified by repeated in vitro and in vivo experiments.

## Prediction of Trastuzumab affinity improvement by computer stimulation

In order to evaluate the accuracy of the prediction of computer simulation, firstly all amino acid sites in the trastuzumab binding region were selected and subjected to virtual mutation, which are mutated into other 19 amino acids in turn, respectively. A PDB file (1N8Z) of the cocrystals of trastuzumab and Her2 was imported into InsightII (accelrys company), CVFF force field was loaded, and hydrogen was added by Biopolymer. Energy minimization was performed on the hydrogen bond while keeping all heavy atoms of the protein fixed to their positions. Energy minimization was performed first by steepest descent method until the maximum derivative is less than 1000 kcal/mol/A and then by conjugate gradient method for total 10,000 steps (with step size of 1fs) to obtain a convergence of 0.01 finally. The optimized structures were obtained and the distance of 6Å away from the antigen was set as contact surface. Water molecules were added at the distance of 25Å around the contact surface. The selected amino acid sites were subjected to amino acid mutation, and based on the rotation isomers library summarized by Ponder and Richards, amino acid molecules at a distance of 6Å from the mutation sites were subjected to auto_rotamer to select the optimal space initiation sites. The water molecules at the peripheral and the antibody molecules out of the contact surface were fixed and subjected to simulated annealing to find the most likely contact mode.

The present invention employed a two-step method to find the possible conformations. The quartic_vdw_no_ Coulomb method was firstly used to select the possible binding conformations, wherein the impact factor of the van der Waals forces in the process was reduced to 0.5 and a 3000-step search was taken for each time, and 60 confirmations were obtained finally. Then, the obtained 60 preliminary conformations were subjected to a 4000-step energy minimization by cell_mutipole method (1 step size = 1fs), wherein the impact factor of the van der Waals and Coulomb force option were set as 0.5, and 50 stages were divided from temperature of 500K to 280K, with 100fs for each stage, and the obtained structures were further subjected to a 8000-step energy minimization. The binding energy, total energy and RMSD of the obtained structures are scored and a most likely structure is picked out to evaluate the binding energy between its different mutants. As shown in table 1, the accuracy of computer prediction reaches 18.2% in recent years.

## Design strategy of improving the affinity of Trastuzumab

First, the contact surface of trastuzumab and Her2 antigen was analyzed: the contact salvation surface of trastuzumab and Her2 antigen is 675Å, which is a relative large contact surface. The amino acids at the peripheral of the contact surface were subjected to virtual mutation in turn. Using the same computer simulation steps mentioned above, 10 mutation sites were selected and predicted to have the maximal improvement and subjected to verification tests.

## Example 1: Cloning of the light and heavy chain constant region genes of human antibodies

Healthy human lymphocytes were isolated with lymphocyte separation medium (Dingguo biotechnology and development Co., Ltd) and total RNA was extracted with TRIZOL Reagent (Invitrogen). According to the sequences disclosed in references (*Cloned human and mouse kappa immunoglobulin constant and J region genes conserve homology in functional segments.* Hieter PA, Max EE, Seidman JG, Maizel JV Jr, Leder P. Cell. 1980 Nov; 22(1 Pt 1): 197-207; and *The nucleotide sequence of a human immunoglobulin C gamma1 gene.* Ellison JW, Berson BJ, Hood LE. Nucleic Acids Res. 1982 Jul 10;10(13):4071-9), the following primers were respectively designed: HC sense: GCTAG CACCA AGGGC CCATC GGTCT TCC; HC antisense: TTTAC CGGGA GACAG GGAGA GGCTC TTC; Lc sense: ACTGT GGCTG CACCA TCTGT CTTCA TCT; Lc antisense: ACACT CTCCC CTGTT GAAGC TCTTT GTG. Genes of the heavy chain constant region and light chain constant region of the antibody were amplified by RT-PCR. The PCR products were purified and recycled by agarose gel electrophoresis and cloned into pGEM-T vector (Promega). The clones were verified to be correct via sequencing. SEQ ID NO: 1 shows the nucleotide sequence of the heavy chain constant region (CH), SEQ ID NO: 2 shows the amino acid sequence of the heavy chain constant region (CH), SEQ ID NO: 3 shows the nucleotide sequence of the light chain constant region (CL) and SEQ ID NO: 4 shows the amino acid sequence of the light chain constant region (CL). The correct clones were designated as pGEM-T/CH and pGEM-T/CL in the present example.

## Example 2: Construction of expression vector of humanized anti-Her2 antibody Trastuzumab

Based on the information and the sequence of the anti-Her2 monoclonal antibody published in PNAS in 1992 (Carter, P. and L. Presta, et al. (1992). Humanization of an anti-p185HER2 antibody for human cancer therapy. Proc Natl Acad Sci U S A 89(10): 4285-9), genes of heavy chain variable region (Her2VH) and light chain variable region (Her2VL) of the anti-human Her2 monoclonal antibody Trastuzumab were synthesized, as shown in Figure 4.

Humanized antibody heavy chain genes were synthesized by overlap PCR using the Her2VH genes and pGEM-T/CH vector as template. The reaction conditions were as follows: 95°C for 15 minutes; 94°C for 50 seconds, 58°C for 50 seconds, 72°C for 50 seconds, 30 cycles; 72°C for 10 minutes. The humanized heavy chain genes contained a restriction enzyme sites Hind III and a signal peptide sequence at the 5 'end and contained a translation termination codon TAA and a restriction enzyme site EcoR I at the 3' end . The signal peptide sequence was: ATG GAT TTT CAG GTG CAG ATT TTC AGC TTC CTG CTA ATC AGT GCC TCA GTC ATA ATA TCC AGA GGA. At last, the PCR products were separated by agarose gel electrophoresis and the target band was recycled and cloned into pGEMT vector, followed by screening positive clones and sequencing. Correct clones verified by sequencing were digested with Hind III and EcoR I. The human antibody heavy chain fragment Her2VHCH was purified and recycled by agarose gel electrophoresis and linked to plasmid pcDNA3.1 (+) (Invitrogen, USA), which was digested with Hind III and EcoR I, to construct a humanized heavy chain eukaryotic expression vector pcDNA3.1(+) (Her2VHCH).

Humanized antibody light chain genes were synthesized by overlap PCR using the Her2VL genes and pGEM-T/CL vector as template. The reaction conditions were as follows: 95°C for 15 minutes; 94°C for 50 seconds, 58°C for 50 seconds, 72°C for 50 seconds, 30 cycles; 72°C for 10 minutes, obtaining the PCR Her2VLCL, which contained a restriction enzyme site Hind III and a signal peptide sequence at the 5' end and contained a translation termination codon TAA and a restriction enzyme site EcoR I at the 3' end. The signal peptide sequence was: ATG GAT TTT CAG GTG CAG ATT TTC AGC TTC CTG CTA ATC AGT GCC TCA GTC ATA ATA TCC AGA GGA. Correct clones verified by sequencing were digested with Hind III and EcoR I. The human antibody light chain fragment Her2VLCL was purified and recycled by agarose gel electrophoresis and linked to plasmid pcDNA3.1 (+) (Invitrogen, USA), which was digested with Hind III and EcoR I, to construct a humanized light chain eukaryotic expression vector pcDNA3.1(+) (Her2VLCL).

## Example 3: Stable expression and purification of the chimeric antibody

$3\times10^5$CHO-K1 cells (ATCC CRL-9618) were inoculated into 3.5cm tissue culture dishes and cultured until reaching 90%-95% confluence before transfection. 10μg of phasmids (including 4μg of phasmid pcDNA3.1 (+) (Her2VHCH) and 6μg of phasmid pcDNA3.1 (Her2VLCL)) and 20μl of Lipofectamine 2000 Reagent (Invitrogen) were dissolved into 500μl of serum-free DMEM medium respectively, and placed for 5 minutes at room temperature. The above two liquid solutions were mixed and incubated for 20 minutes at room temperature to form a DNA-liposome complex, during which the serum-containing medium in the petri dishes was replaced with 3ml of non-serum DMEM medium. Then, the formed DNA-liposome complex was added into a plate and incubated for 4 hours in a $CO_2$ couveuse, and then supplemented with 2ml of DMEM complete medium containing 10% serum and still incubated in the $CO_2$ couveuse. After 24 hours of transfection, the cells were cultured in selective medium containing 600μg/ml of G418 to select resistant clones. detecting The cell culture supernatant was detected by ELISA to select high-expression clones: An ELISA plate was coated with goat anti-human IgG (Fc) and placed overnight at 4°C, then blocked with 2% BSA-PBS for 2 hours at 37°C; added with the resistant clone culture supernatant to be tested or standard samples (Human myeloma IgG1, κ) and warm incubated for 2 hours at 37°C; added with HRP-goat anti-human IgG (κ) for binding reaction and warm incubated for 1 hour at 37°C; added with TMB and reacted for 5 minutes at 37°C; and added with $H_2SO_4$ to terminate the reaction finally. And the A450 values were measured. The selected high expression clones were cultured with serum-free medium for amplification. The humanized antibody trastuzumab was separated and purified by Protein A affinity column (GE). The purified antibody was subjected to dialysis with PBS. And finally, the concentration of the purified antibody was quantitatively determined by UV absorption.

## Example 4: Construction and expression of the trastuzumab antibody mutants

Trastuzumab antibody mutants were constructed by overlap PCR and the methods of construction, expression and purification of the trastuzumab antibody mutants were similar to that of trastuzumab humanized antibody. Ten trastuzumab antibody mutants were constructed and named as Hmut 1 to Hmut 10. The amino acid sequences are shown as SEQ ID NO: 5 ~ SEQ ID NO: 24 respectively.

## Example 5: ELISA identification of the trastuzumab mutants

Her2 extracellular proteins were expressed and purified according to the method disclosed by Carter, then coated onto a ELISA plate and incubated for 2 hours at 37°C. Then, antibodies with a fixed concentration and the Her2 ectodomain proteins diluted at geometric proportion were co-incubated for 1 hour at room temperature. And the affinity level was calculated by identifying the amount of free antibody in the incubated antibody-antigen complexes. For details, refer to: (Carter P, et al. (1992) Humanization of an anti-p185HER2 antibody for human cancer therapy. Pro Natl Acad Sci USA 89: 4285-4289*;* Friguet B, Chaffotte AF, Djavadi-Ohaniance L, Goldberg ME (1985) Measurements of the true affinity constant in solution of antigen-antibody complexes by enzyme-linked immunosorbent assay. J Immunol Methods 77:305-319*)*. As a result, six mutation sites in the ten experimental groups showed the improvement of affinity and the accuracy reached 60%. Wherein, four mutation sites capable of improving trastuzumab affinity proved by experiments were shown in Fig.3.

Table 1 Prediction and experimental results of the antibody affinity of trastuzumab

| Site | Mutation site | $Kd^{WT}/Kd^{mutant}$ |
|---|---|---|
| | $KD^{WT}$ = 0.16 ± 0.02 nM | |
| L28Asp | Pro | 0.83 ± 0.12 |
| L28Asp | Met | 0.32 ± 0.07 |
| L30Asn | Ser | ND |
| L30Asn | Arg | 1.74 ± 0.17 |
| L32Ala | Gln | ND |
| L94Thr | Tyr | 0.87 ± 0.05 |
| H55Asn | Lys | 2.01 ± 0.19 |
| H55Asn | Pro | 0.08 ± 0.01 |
| H57Tyr | Ile | 0.06 ± 0.01 |
| H102Asp | Met | 0.54 ± 0.15 |
| H103Lys | Arg | 0.04 ± 0.01 |

SD: experimental error, deriving from three independent experiments; WT: trastuzumab antibody; ND: the affinity is too weak to be detected.

Table 2. Affinity of the antibody mutants detected by competitive ELISA

| Name of mutant | Site | mutated amino acid | KdWT/Kdmutant | KdHerc/Kdmutant |
|---|---|---|---|---|
| | KdWT = 0.16 ± 0.02 nM | KdHerc = 0.21 ± 0.04 nM | | |
| Hmut1 | L28Asp | Arg | 1.86 ± 0.09 | 2.44 ± 0.12 |
| Hmut2 | L28Asp | Pro | 0.83 ± 0.12 | 1.09 ± 0.16 |
| Hmut3 | L93Thr | Tyr | 1.64 ± 0.18 | 2.15 ± 0.24 |
| Hmut4 | L93Thr | Asn | 0.21 ± 0.08 | 0.28 ± 0.11 |
| Hmut5 | H55Asn | Pro | 0.08 ± 0.01 | 0.11 ± 0.01 |
| Hmut6 | H55Asn | Lys | 2.01 ± 0.19 | 2.64 ± 0.25 |
| Hmut7 | H59Arg | Lys | 0.75 ± 0.02 | 0.98 ± 0.03 |
| Hmut8 | H102Asp | Thr | 2.16 ± 0.16 | 2.84 ± 0.21 |
| Hmut9 | H102Asp | Tyr | 3.11 ± 0.28 | 4.09 ± 0.37 |
| Hmut10 | H102Asp | Lys | 2.31 ± 0.20 | 3.03 ± 0.26 |

SD: experimental error, deriving from three independent experiments; WT: un-mutated antibody sequence; Hrec: commercially available Herceptin.

**Experiment of improving the antibody affinity of Rituximab**

Rituximab is a human-mouse chimeric monoclonal antibody consisting of mouse Fab and human Fc produced by genetic engineering, with molecular weight of about 150kDa. Rituximab binds specifically to the CD20 antigens on B lymphocytes, and eventually causes the death of B lymphocytes. It is used for the treatment of non-Hodgkin lymphomas.

Methods of site-directed mutagenesis of Rituximab

Strategy of antibody mutation of Rituximab

At first, the contact surface on rituximab was analyzed. Usually the solvent accessible surface (SAS) between a short peptide and a protein is about 400-700Å, which is usually smaller than the solvent accessible surface between a protein and a protein. And the SAS between rituximab and short peptide CD20 is 440Å, which is considered to be a relatively small SAS between the interaction of short peptides and proteins. The amino acids at the periphery the contact surface were selected and subjected to virtual mutation in turn.

A PDB file of the cocrystal of rituximab and CD20 antigen was imported into InsightII (Accelrys), CVFF force field was loaded,and hydrogen was added by Biopolymer. A 1000-step energy minimization was performed on the hydrogen bond while keeping all heavy atoms of the protein fixed to their positions ( with step size of 1fs), to obtain a convergence of 0.01 finally. The optimized structures were obtained and the distance of 6Å away from the antigen was set as contact surface. Water molecules were added at the distance of 25Å around the contact surface. The selected amino acid sites were subjected to amino acid mutation, and based on the rotation isomers library summarized by Ponder and Richards, amino acid molecules at a distance of 6Å from the mutation sites were subjected to auto_rotamer to select the optimal space initiation sites. The water molecules at the peripheral and the antibody molecules out of the contact surface were fixed and subjected to simulated annealing to find the most likely contact mode.

The present invention employed a two-step method to find the possible conformations. The quartic_vdw_no_Coulomb method was firstly used to select the possible binding conformations, wherein the impact factor of the van der Waals forces in the process was reduced to 0.5 and a 3000-step search was taken for each time, and 60 confirmations were obtained finally. Then, the obtained 60 preliminary conformations were subjected to a 4000-step energy minimization by cell_mutipole method (1 step size = 1fs), wherein the impact factor of the van der Waals and Coulomb force option were set as 0.5, and 50 stages were divided from temperature of 500K to 280K, with 100fs for each stage, and the obtained structures were further subjected to a 8000-step energy minimization. The structures produced in the above-mentioned process was subjected to RMSD (Root mean square deviation) analysis and the conformation changes (heavy atom) between the amino acids on the antigen peptide of the structural complex, binding tightly to the antibody and the amino acids before being mutated were compared. And finally, those structures with minimized total energy and relative less RMSD were selected in the present invention.

The selected structures were imported into charmm for energy minimization. MM-PBSA method was used to evaluate the energy. In order to evaluate the accuracy of computer prediction, the present inventors selected the amino acids that were predicted to have an improved affinity and the amino acids that were predicted to have a reduced affinity respectively at three candidate sites for verification tests.

**Construction of Rituximab antibody**

**Example 6: Gene clone of the light and heavy chain constant region of human antibodies**

Healthy human lymphocytes were isolated with lymphocyte separation medium (Dingguo biotechnology and development Co., Ltd) and total RNA was extracted with TRIZOL Reagent (Invitrogen). According to the sequences disclosed in references (*Cloned human and mouse kappa immunoglobulin constant and J region genes conserve homology in functional segments.* Hieter PA, Max EE, Seidman JG, Maizel JV Jr, Leder P. Cell. 1980 Nov; 22(1 Pt 1): 197-207*;* and *The nucleotide sequence of a human immunoglobulin C gamma1 gene.* Ellison JW, Berson BJ, Hood LE. Nucleic Acids Res. 1982 Jul 10; 10(13):4071-9)*,* the following primers were respectively designed: HC sense: GCTAG CACCA AGGGC CCATC GGTCT TCC; HC antisense: TTTAC CGGGA GACAG GGAGA GGCTC TTC; Lc sense: ACTGT GGCTG CACCA TCTGT CTTCA TCT; Lc antisense: ACACT CTCCC CTGTT GAAGC TCTTT GTG. Genes of the heavy chain constant region and light chain constant region of the antibody were amplified by RT-PCR. The PCR products were purified and recycled by agarose gel electrophoresis and cloned into pGEM-T vector. The clones were verified to be correct via sequencing. SEQ ID NO: 1 and SEQ ID NO: 2 show the nucleotide sequence and amino acid sequence of the heavy chain constant region (CH) respectively. SEQ ID NO: 3 and SEQ ID NO: 4 show nucleotide sequence and amino acid sequence of the light chain constant region (CL) respectively. The correct clones were designated as pGEM-T/CH and pGEM-T/CL in the present example.

**Example 7: Construction of the expression vector of anti-CD20 chimeric antibody Rituximab**

Genes of heavy chain variable region (C2B8VH) and light chain variable region gene (C2B8VL) of the anti-human CD20 monoclonal antibody Rituximab (C2B8) were synthesized with reference to the information and sequences of the anti-human CD20 monoclonal antibody disclosed in the U.S. Patent 6,399,061. Fig. 6 shows nucleotide sequence and amino acid sequence of the C2B8 heavy chain variable region and light chain variable region.

Humanized antibody heavy chain genes were synthesized by overlap PCR using the C2B8VH genes and pGEM-T/CH vector as template. The reaction conditions were as follows: 95°C for 15 minutes; 94°C for 50 seconds, 58°C for 50 seconds, 72°C for 50 seconds, 30 cycles; 72°C for 10 minutes. The humanized heavy chain genes contained a restriction enzyme site Hind III and a signal peptide sequence at the 5' end and contained a translation termination codon TAA and a restriction enzyme site EcoR I at the 5' end. The sequence of the signal peptide was: ATG GGA TTC AGC AGG ATC TTT CTC TTC CTC CTG TCA GTA ACT ACA GGT GTC CAC TCC. At last, the PCR products were separated by agarose gel electrophoresis and the target band was recycled and cloned into the pGEMT vector, followed by screening positive clones and sequencing. Correct clones verified by sequencing were digested with Hind III and EcoR I. The human antibody heavy chain fragment C2B8VHCH was purified and recycled by agarose gel electrophoresis and linked to plasmid pcDNA3.1 (+) (Invitrogen, USA), which was digested with Hind III and EcoR I, to construct a humanized heavy chain eukaryotic expression vector pcDNA3.1 (+) (C2B8VHCH).

Humanized antibody light chain genes were synthesized by overlap PCR using the C2B8VL genes and pGEM-T/CL vector as template. The reaction conditions were as follows: 95°C for 15 minutes; 94°C for 50 seconds, 58°C for 50 seconds, 72°C for 50 seconds, 30 cycles; 72°C for 10 minutes, obtaining the PCR product C2B8VLCL, which contained a restriction enzyme site Hind III and a signal peptide sequence at the 5 'end and contained a translation termination codon TAA and a restriction enzyme site EcoR I at the 3' end. The sequence of the signal peptide was: ATG GAT TTT CAA GTG CAG ATT TTC AGC TTC CTG CTA ATC AGT GCT TCA GTC ATA ATG TCC AGA GGA. Correct clones verified by sequencing were digested with Hind III and EcoR I. The human antibody light chain fragment C2B8VLCL was purified and recycled by agarose gel electrophoresis and linked to plasmid pcDNA3.1(+) (Invitrogen, USA),which was digested with Hind III and EcoR I, to construct a humanized light chain eukaryotic expression vector pcDNA3.1 (C2B8VLCL).

## Example 8: Stable expression and purification of the chimeric antibody

$3\times10^5$ CHO-K1 cells (ATCC CRL-9618 ) were incubated into 3.5cm tissue culture dishes and cultured until reaching 90%-95% confluence before transfection. 10μg of phasmids (including 4μg of phasmid pcDNA3.1 (+) (C2B8VHCH) and 6μg of phasmid pcDNA3.1 (C2B8VLCL)) and 20μl of Lipofectamine 2000 Reagent (Invitrogen) were dissolved into 500μl of serum-free DMEM medium respectively, and placed for 5 minutes at room temperature. The above two liquid solutions were mixed and incubated for 20 minutes at room temperature to form a DNA-liposome complex, during which the serum-containing medium in the petri dishes was replaced with 3ml of non-serum DMEM medium. Then, the formed DNA-liposome complex was added into a plate and incubated for 4 hours in a $CO_2$ couveuse, and then supplemented with 2ml of DMEM complete medium containing 10% serum and still incubated in the $CO_2$ couveuse. After 24 hours of transfection, the cells were cultured in selective medium containing 600μg/ml of G418 to select resistant clones. detecting The cell culture supernatant was detected by ELISA to select high-expression clones: An ELISA plate was coated with goat anti-human IgG (Fc) and placed overnight at 4°C, then blocked with 2% BSA-PBS for 2 hours at 37°C; added with the resistant clone culture supernatant to be tested or standard samples (Human myeloma IgG1, κ) and warm incubated for 2 hours at 37°C; added with HRP-goat anti-human IgG (κ) for binding reaction and warm incubated for 1 hour at 37°C; added with TMB and reacted for 5 minutes at 37°C; and added with $H_2SO_4$ to terminate the reaction finally. And the A450 values were measured. The selected high expression clones were cultured with serum-free medium for amplification. The chimeric antibody C2B8 was separated and purified by Protein A affinity column (GE). The purified antibody was subjected to dialysis with PBS. And finally, the concentration of the purified antibody was quantitatively determined by UV absorption.

## Example 9: Construction and expression of the C2B8 antibody mutants

C2B8 antibody mutants were constructed by overlap PCR and the methods of construction, expression and purification of the C2B8 antibody mutant were similar to that of the C2B8 chimeric antibody. Ten C2B8 antibody mutants were constructed and named as Rmut1 to Rmut7. Their amino acid sequences are shown as SEQ ID NO: 25 ~ SEQ ID NO: 38 respectively.

## Example 10: Biacore identification of Rituximab and its mutants

A SA chip was balanced in 50μl/min of PBS solution for 30 minutes at 25°C and then activated three times with activation solution of 1M NaCl and 50mM NaOH, for 1minute per time. Biotin labeled antigen peptide (a fragment of the CD20 extracellular domain, see to "Structural Basis for Recognition of CD20 by Therapeutic Antibody Rituximab. Du, J.; Wang, H.; Zhong, C. (...). J Biol Chem, 2007, 282(20): 15073-15080″) was diluted to a final concentration of 1μg/ml and used to coat the chip at flow rate of 10μl/min. ΔRu was 1000. Then the chip was balanced in 50μl/min of PBS solution for 10 minutes. The balanced SA chip was blocked with 0.04% of biotin solution. The antibody was diluted to five concentrations by double dilution. The samples were loaded at the flow rate of 50μl/min for 75 seconds and dissociated with PBS solution for 10 minutes. Fig.7 shows the sensorgram detected by biacore at the same sample concentration. The detailed affinity values were shown in table 3. As a result, the affinity of C2B8 antibody mutant Rmut3 was improved by 6.08 times and the affinity of C2B8 antibody mutant Rmut7 was improved by 3.96 times. The accuracy of prediction reached 71.4%. As shown in Figure 5, the mutation sites that showed the improved affinity were Asp at site 57 and Tyr at site 102 of the heavy chain.

Table 3 Affinity of the antibody mutants detected by biacore

| Name | Mutation site and mutated amino acid | KdWT/Kdmutant | KdRitu/Kdmutant |
|---|---|---|---|
| | $K_d^{WT}$=44.1 ± 0.30 nM | $K_d^{ritu}$=56.1 ± 0.40 nM | |
| Rmut1 | H55NE | 0.54±0.21 | 0.69±0.27 |
| Rmut2 | H55NR | 0.61±0.18 | 0.78±0.23 |
| Rmut3 | H57DE | 6.08±1.48 | 7.73±1.88 |
| Rmut4 | H102YR | 1.75±0.25 | 2.23±0.32 |
| Rmut5 | H102YS | 1.85±0.35 | 2.35±0.45 |
| Rmut6 | H102YT | 1.84±0.24 | 2.34±0.31 |
| Rmut7 | H102YK | 3.96±0.39 | 5.04±0.50 |

ND: not detected by biacore; WT: un-mutated C2B8; Ritu: commercially available rituximab.

**Experiment of improving the affinity of CTLA4-Ig fusion receptor**

Cytotoxic T-lymphocyte antigen 4 (CTLA-4) is a homologous dimmers mainly expressed in activated T cells, which is highly homologous with CD28.

Abatacept is a fusion protein of CTLA-4 extracellular domain with an immunoglobulin, which inhibits the activation of T cell by binding to B7 molecule and thus is used as a specific co-stimulatory modulator for the treatment of rheumatoid arthritis refractory that did not response to anti-TNFα therapy. Belatacept was also developed by Bristol-Myers Squibb. It differs from abatacept (Orencia) by only 2 amino acids, but it improves the affinity to ligands (CD80, CD86) significantly.

Experiment methods of CTLA4/Ig site-directed mutation

A PDB file (1i85) of the cocrystals of CTLA4/Ig and CD86 was imported into InsightII (Accelrys), CVFF force field was loaded, and hydrogen was added by Biopolymer. Energy minimization was performed on the hydrogen bond while keeping all heavy atoms of the protein fixed to their positions. Energy minimization was performed first by steepest descent method until the maximum derivative is less than 1000 kcal/mol/A and then by conjugate gradient method for total 10,000 steps (with step size of 1fs) to obtain a convergence of 0.01 finally. The optimized structures were obtained and the distance of 6Å away from the antigen was set as contact surface. Water molecules were added at the distance of 25Å around the contact surface. The selected amino acid sites were subjected to amino acid mutation, and based on the rotation isomers library summarized by Ponder and Richards, amino acid molecules at a distance of 6Å from the mutation sites were subjected to auto_rotamer to select the optimal space initiation sites. The water molecules at the peripheral and the antibody molecules out of the contact surface were fixed and subjected to simulated annealing to find the most likely contact mode.

The present invention employed a two-step method to find the possible conformations. The quartic_vdw_no_Coulomb method was firstly used to select the possible binding conformations, wherein the impact factor of the van der Waals forces in the process was reduced to 0.5 and a 3000-step search was taken for each time, and 60 confirmations were obtained finally. Then, the obtained 60 preliminary conformations were subjected to a 4000-step energy minimization by cell_mutipole method (1 step size = 1fs), wherein the impact factor of the van der Waals and Coulomb force option were set as 0.5, and 50 stages were divided from temperature of 500K to 280K, with 100fs for each stage, and the obtained structures were further subjected to a 8000-step energy minimization. The binding energy, total energy and RMSD of the obtained structures are scored and a most likely structure is picked out to evaluate the binding energy of the different mutants. In order to evaluate the accuracy of computer prediction, the present inventors selected the amino acids that were predicted to have an improved affinity at three candidate sites for verification tests.

Construction and functional detection of the CTLA4/Ig mutants

**Example 11: Cloning of the genes of CTLA-4 extracellular domain and Fc region**

Healthy human lymphocytes were isolated with lymphocyte separation medium and the total RNA was extracted with TRIZOL Reagent (Invitrogen Co.,Ltd). Primers were designed to amplify the genes of the CTLA-4 extracellular domain (Gene ID: 1493) and the Fc region of the antibody was amplified by Hot Start PCR using the following primers: FC sense: GCCCAGATTCTGATCAGGAGCCCAAATCTTCTGAC; and FC antisense: GAATTCTCATTTACCCGGAGACAGG. The reaction conditions were as follows: 94°C for 15 minutes; 94°C for 45 seconds, 60°C for 45 seconds, 72°C for 1 minute and 10 seconds, 30 cycles; 72°C for 10 minutes. The PCR products were purified and recycled by agarose gel electrophoresis and cloned into pGEM-T(promega) vector. The clones were verified to be correct via sequencing. Fig.8 shows the nucleotide sequence and amino acid sequence of the CTLA-4. SEQ ID NO:39 and SEQ ID NO:40 show the nucleotide sequence and amino acid sequence of the Fc region, respectively. The correct clones were designated as pGEM-T/T and pGEM-T/Fc in the present example.

**Example 12: Construction of expression vector of the CTLA-4/Ig fusion protein**

The synthetic signal peptide sequence of SEQ ID NO: 41 and the cloned CTLA-4 extracellular gene fragment were subjected to overlap PCR with designed primers. Correct fragment verified by sequencing and the Fc fragment of the antibody were subjected to overlap PCR and the resultant product was linked into pGEM-T vector for sequencing. Correct clones of the CTLA-4/Ig fusion protein were digested with Hind III and EcoR I, and purified and recycled by agarose gel electrophoresis and linked to plasmid pcDNA3.1 (+) (Invitrogen Ltd., USA), which was digested with Hind III and EcoR I, to construct a humanized heavy chain eukaryotic expression vector pcDNA3.1 (+), designated as pcDNA3.1 (+)(CTLA-4/Ig).

**Example 13: Stable expression and purification of fusion receptor**

$3\times10^5$CHO-K1 cells (ATCC CRL-9618) were inoculated into 3.5cm tissue culture dishes and cultured until reaching 90%-95% confluence before transfection. 10μg of phasmids (10μg of phasmid pcDNA3.1(+) (CTLA-4/Ig)) and 20μl of Lipofectamine 2000 Reagent (Invitrogen) were dissolved into 500μl of serum-free DMEM medium respectively, and placed for 5 minutes at room temperature. The above two liquid solutions were mixed and incubated for 20 minutes at room temperature to form a DNA-liposome complex, during which the serum-containing medium in the petri dishes was replaced with 3ml of non-serum DMEM medium. Then, the formed DNA-liposome complex was added into a plate and incubated for 4 hours in a $CO_2$ couveuse, and then supplemented with 2ml of DMEM complete medium containing 10% serum and still incubated in the $CO_2$ couveuse. After 24 hours of transfection, the cells were cultured in selective medium containing 600μg/ml of G418 to select resistant clones. detecting The cell culture supernatant was detected by ELISA to select high-expression clones: An ELISA plate was coated with goat anti-human IgG (Fc) and placed overnight at 4°C, then blocked with 2% BSA-PBS for 2 hours at 37°C; added with the resistant clone culture supernatant to be tested or standard samples (Abatacept) and warm incubated for 2 hours at 37°C; added with HRP-goat anti-human Fc (CH2) for binding reaction and warm incubated for 1 hour at 37°C; added with TMB and reacted for 5 minutes at 37°C; and added with $H_2SO_4$ to terminate the reaction finally. And the A450 values were measured. The selected high expression clones were cultured with serum-free medium for amplification. The chimeric antibody C2B8 was separated and purified by Protein A affinity column (GE). The purified antibody was subjected to dialysis with PBS and quantified by UV absorption.

**Example 14: Construction and expression of the fusion antibody mutants**

The CTLA-4/Ig mutants were constructed by overlap PCR and the methods of construction (as shown in Fig. 8), expression and purification of the CTLA-4/Ig mutants were similar to that of CTLA-4/Ig fusion protein. The amino acid sequences of the mutants are shown as SEQ ID NO:42~SEQ ID NO:50.

**Example 15: Biacore indentification of Abatacept and CTLA-4/Ig mutants**

A CM5 chip was balanced in 50μl/min of PBS solution for 30 minutes at 25°C and then activated for 8 minutes with a mixture of 100μl of N-Hydroxysulfosuccinimide (NHS) and 100μl of 1-ethyl-3-(3-dimethyl-amino propyl)-carbodiimide (EDC) at the flow rate of 10μl/ml. The CM5 chip was coated with CD86-Fc protein (R&D) at a flow rate of 10μl/ml and the final ΔRu=1000. Then the chip was balanced in the PBS buffer for 10 minutes. The samples to be tested were diluted to five concentrations by double dilution. The diluted samples were loaded at a flow rate of 50μl/min for 75 seconds and dissociated with PBS solution for 10 minutes. Fig.9 shows the sensorgram detected by biacore at the same sample concentration. The detailed affinity values are shown in table 4. Wherein, the affinity of CTLA-4Ig constructed according to the present invention was similar to the affinity of Abatacept. Single site mutants with higher improved affinity were as follows: CTmut1 and CTmut2 mutants, the affinity of which were improved by 4.04 times and 3.98 times respectively; mutant CTmut6, the affinity of which was improved by 2.29 times; and mutant CTmut10, the affinity of which was improved by 2.68 times. As a result, the accuracy of the prediction reached 70%.

Table 4

| Mutant No. | Site | Mutated to amino acid | $K_d^{WT}/K_d^{mutant}$ | $K_d^{abat}/K_d^{mutant}$ |
|---|---|---|---|---|
| | $K_d^{WT}$=44.1 ± 0.30 | nM $K_d^{ritu}$=56.1 ± 0.40 nM | | |
| CTmut1 | D31Ala | Tyr | 3.98 ± 0.19 | 4.24 ± 0.20 |
| CTmut2 | D31Ala | Lys | 4.04 ± 0.90 | 4.31 ± 0.96 |
| CTmut3 | D53Thr | Lys | 0.55 ± 0.14 | 0.58 ± 0.15 |
| CTmut4 | D55Met | Glu | 1.75 ± 0.07 | 1.87 ± 0.08 |
| CTmut5 | D63Leu | Lys | 1.85 ± 0.16 | 1.97 ± 0.17 |
| CTmut6 | D63Leu | Tyr | 2.29 ± 0.31 | 2.44 ± 0.33 |
| CTmut7 | D35Arg | Pro | 0.55 ± 0.14 | 0.58 ± 0.15 |
| CTmut8 | D106Leu | Glu | 2.00 ± 0.39 | 2.13 ± 0.42 |
| CTmut9 | D106Leu | Asn | 0.88 ± 0.06 | 0.94 ± 0.06 |
| CTmut10 | D106Leu | Ser | 2.68 ± 1.14 | 2.86 ± 1.22 |

SD: experimental error, determined by three independent experiments; WT: un-mutated origin fusion receptor; abat: commercially available abatacept.

## INDUSTRIAL APPLICABILITY

The method according to the present invention can be widely used to improve the affinity between proteins to facilitate the development of the proteins with biological and medical significance. Meanwhile, the combination of antibody evolution law and computer simulation techniques proposes a new concept for the future computer-aided design.

## Claims

**1.** A method of acquiring antibodies or proteins with high affinity by computer-aided design, comprising the steps of:

1) based on a known structure of a cocrystal of a complex of an antibody or a protein molecule, determining candidate sites of virtual mutation of the antibody or the protein molecule;
2) simulating amino acid mutations in candidate sites of virtual mutation in turn by computer so as to acquire preliminary optimized molecular structures;
3) searching out conformations of the preliminary optimized molecular structures by computer, so as to acquire simulated structures of the antibody or the protein molecule after virtual mutation;
4) analyzing total energies and root mean square deviations of the optimized structures of the antibody or the protein molecular, and selecting mutant conformations with minimized energy and less root mean square deviations to analyze binding energies binding to the protein molecule and to acquire simulative structures; and
5) based on the simulative structures, constructing and predicting mutants of the antibody or the protein with improved affinity, and validating the improved affinity by experiments so as to acquire an antibody mutant or a protein mutant with high affinity.

**2.** According to the method of claim 1, wherein, in step 1), based on the known characteristic changes on the structure of the cocrystal during affinity maturation of the antibody or protein, determining the virtual mutation sites; and selecting the amino acids that are biased distributed on the surface and contact surface of the complex as candidate mutated amino acids.

**3.** According to the method of claim 2, wherein based on the structure of the cocrystal of the complex of the antibody or a protein molecule, selecting said mutation sites of step 1); the selected mutation sites locating at the periphery of the contact surface between an antibody or protein molecule and an antigen or binding protein, without interacting with the antigen or binding protein.

**4.** According to the method of claim 2, wherein in step 2), said virtual mutation sites are mutated into an amino acid selected from the group consisting of Glu, Arg, Asn, Ser, Thr, Tyr, Lys, Asp, Pro and/or Ala.

**5.** According to the method of claim 1, wherein said step 4) comprises the steps of:

a) sorting the preliminary optimized antibody or protein molecule of step 3) according to the overall energy;
b) based on the cocrystal structure of complex of the antibody or protein molecule complex, determining key amino acids involved in binding on the target molecule;
c) mutating the key amino acids involved in binding, simulating the optimized structures and crystal structures and analyzing the root mean square deviations, selecting the mutant structures with minimized total energies and less root mean square deviations to calculate, analyze and sort their binding energies;
d) based on the sorting results of step c), acquiring the simulative structures with high affinity of the antibody or the protein molecule.

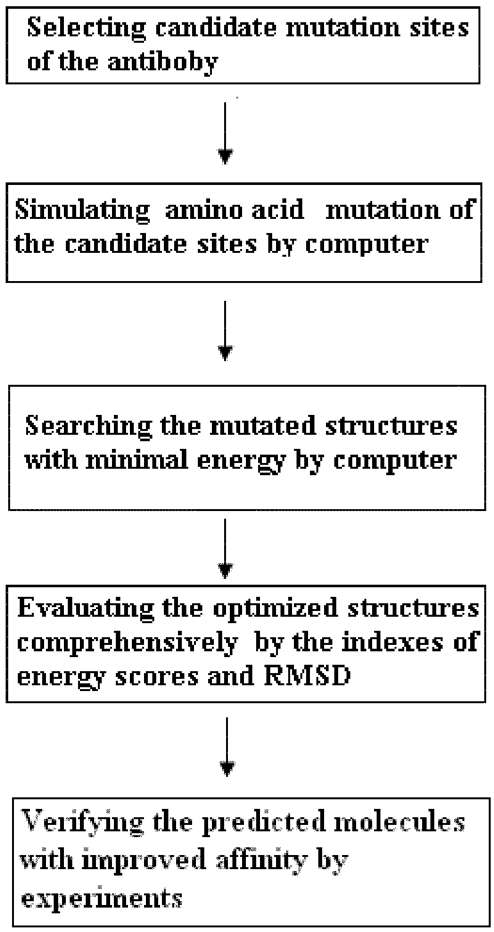
Selecting candidate mutation sites of the antiboby
Simulating amino acid mutation of the candidate sites by computer
Searching the mutated structures with minimal energy by computer
Evaluating the optimized structures comprehensively by the indexes of energy scores and RMSD
Verifying the predicted molecules with improved affinity by experiments

Fig. 1

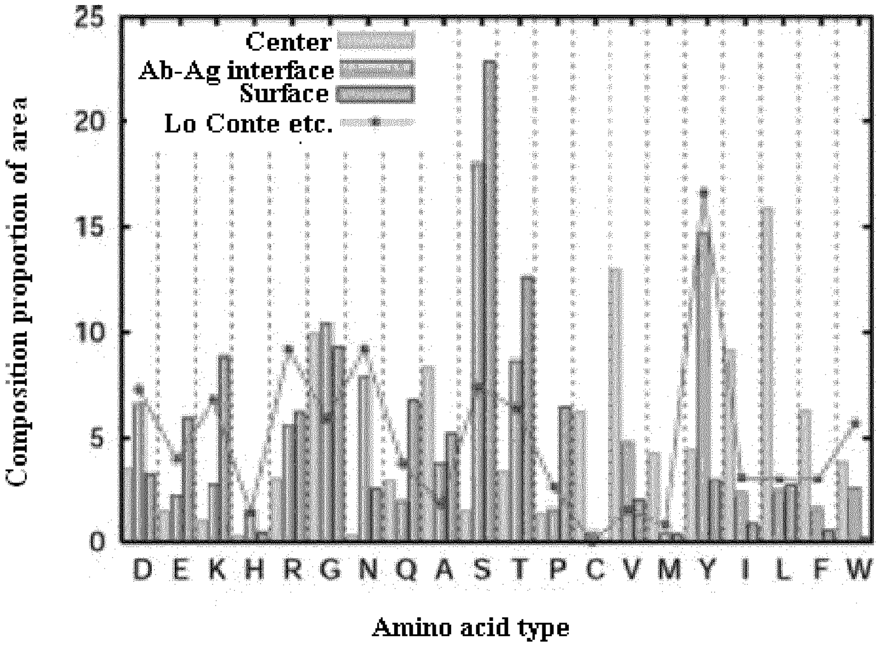
Center
Ab-Ag interface
Surface
Lo Conte etc.
Composition proportion of area
0
5
10
15
20
25
D E K H R G N Q A S T P C V M Y I L F W
Amino acid type

Fig. 2

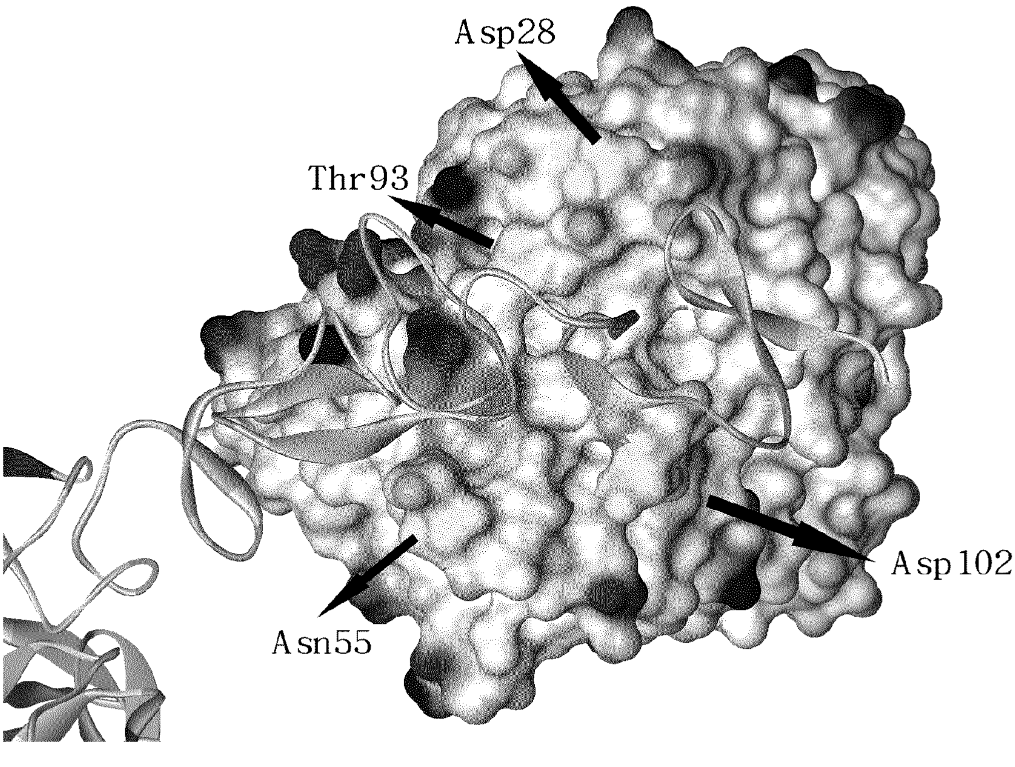
Asp28
Thr93
Asp102
Asn55

Fig. 3

## Trastuzumab VH

```
                 10        20        30        40        50        60
1       GAGGTTCAGCTGGTGGAGTCTGGCGGTGGCCTGGTGCAGCCAGGGGGCTCACTCCGTTTG
1        E  V  Q  L  V  E  S  G  G  G  L  V  Q  P  G  G  S  L  R  L

                 70        80        90       100       110       120
61      TCCTGTGCAGCTTCTGGCTTCAACATTAAAGACACCTATATACACTGGGTGCGTCAGGCC
21       S  C  A  A  S  G  F  N  I  K  D  T  Y  I  H  W  V  R  Q  A

                130       140       150       160       170       180
121     CCGGGTAAGGGCCTGGAATGGGTTGCAAGGATTTATCCTACGAATGGTTATACTAGATAT
41       P  G  K  G  L  E  W  V  A  R  I  Y  P  T  N  G  Y  T  R  Y

                190       200       210       220       230       240
181     GCCGATAGCGTCAAGGGCCGTTTCACTATAAGCGCAGACACATCCAAAAACACAGCCTAC
61       A  D  S  V  K  G  R  F  T  I  S  A  D  T  S  K  N  T  A  Y

                250       260       270       280       290       300
241     CTGCAGATGAACAGCCTGCGTGCTGAGGACACTGCCGTCTATTATTGTTCTAGATGGGGA
81       L  Q  M  N  S  L  R  A  E  D  T  A  V  Y  Y  C  S  R  W  G

                310       320       330       340       350       360
301     GGGGACGGCTTCTATGCTATGGACTACTGGGGTCAAGGAACCCTGGTCACCGTCTCCTCG
101      G  D  G  F  Y  A  M  D  Y  W  G  Q  G  T  L  V  T  V  S  S
```

## Trastuzumab VL

```
                 10        20        30        40        50        60
1       GACATCCAGATGACCCAGTCCCCGAGCTCCCTGTCCGCCTCTGTGGGCGATAGGGTTACC
1        D  I  Q  M  T  Q  S  P  S  S  L  S  A  S  V  G  D  R  V  T

                 70        80        90       100       110       120
61      ATCACCTGCCGTGCCAGTCAGGATGTGAATACTGCTGTAGCCTGGTATCAACAGAAACCA
21       I  T  C  R  A  S  Q  D  V  N  T  A  V  A  W  Y  Q  Q  K  P

                130       140       150       160       170       180
121     GGAAAAGCTCCGAAACTACTGATTTACTCGGCATCCTTCCTCTACTCTGGAGTCCCTTCT
41       G  K  A  P  K  L  L  I  Y  S  A  S  F  L  Y  S  G  V  P  S

                190       200       210       220       230       240
181     CGCTTCTCTGGCTCCAGATCTGGGACGGATTTCACTCTGACCATCAGCAGTCTGCAGCCG
61       R  F  S  G  S  R  S  G  T  D  F  T  L  T  I  S  S  L  Q  P

                250       260       270       280       290       300
241     GAAGACTTCGCAACTTATTACTGTCAGCAACATTATACTACTCCTCCCACGTTCGGACAG
81       E  D  F  A  T  Y  Y  C  Q  Q  H  Y  T  T  P  P  T  F  G  Q

                310       320
301     GGTACCAAGGTGGAGATCAAA
101      G  T  K  V  E  I  K
```

Fig. 4

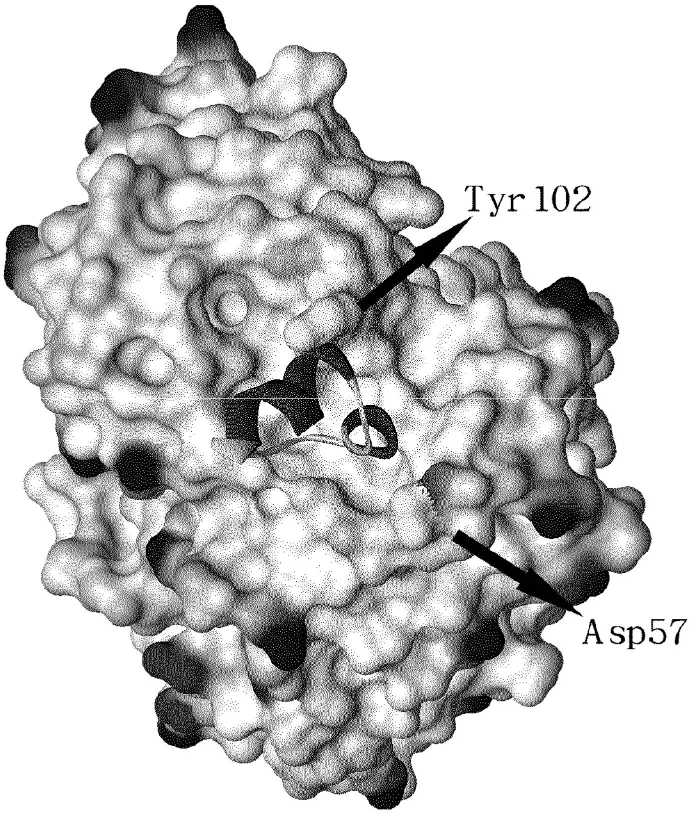


Fig. 5

Rituximab VH

```
                10        20        30        40        50        60
1       CAGGTACAACTACAGCAGCCTGGGGCTGAGCTGGTGAAGCCTGGGGCCTCAGTGAAGATG
1        Q  V  Q  L  Q  Q  P  G  A  E  L  V  K  P  G  A  S  V  K  M

                70        80        90       100       110       120
61      TCCTGCAAGGCTTCTGGCTACACATTTACCAGTTACAATATGCACTGGGTAAAGCAGACA
21       S  C  K  A  S  G  Y  T  F  T  S  Y  N  M  H  W  V  K  Q  T

               130       140       150       160       170       180
121     CCTGGTCGGGGCCTGGAATGGATTGGAGCTATTTATCCAGGAAATGGTGATACTTCCTAC
41       P  G  R  G  L  E  W  I  G  A  I  Y  P  G  N  G  D  T  S  Y

               190       200       210       220       230       240
181     AATCAGAAGTTCAAGGGCAAGGCCACACTGACTGCAGACAAATCCTCCAGCACAGCCTAC
61       N  Q  K  F  K  G  K  A  T  L  T  A  D  K  S  S  S  T  A  Y

               250       260       270       280       290       300
241     ATGCAGCTCAGCAGCCTGACATCTGAAGACTCTGCGGTCTATTACTGTGCAAGATCGACT
81       M  Q  L  S  S  L  T  S  E  D  S  A  V  Y  Y  C  A  R  S  T

               310       320       330       340       350       360
301     TACTACGGCGGTGACTGGTACTTCAATGTCTGGGGCGCAGGGACCACGGTCACCGTCTCT
101      Y  Y  G  G  D  W  Y  F  N  V  W  G  A  G  T  T  V  T  V  S

361     GCA
121      A
```

Rituximab VL

```
                10        20        30        40        50        60
1       CAAATTGTTCTCTCCCAGTCTCCAGCAATCCTGTCTGCATCTCCAGGGGAGAAGGTCACA
1        Q  I  V  L  S  Q  S  P  A  I  L  S  A  S  P  G  E  K  V  T

                70        80        90       100       110       120
61      ATGACTTGCAGGGCCAGCTCAAGTGTAAGTTACATCCACTGGTTCCAGCAGAAGCCAGGA
21       M  T  C  R  A  S  S  S  V  S  Y  I  H  W  F  Q  Q  K  P  G

               130       140       150       160       170       180
121     TCCTCCCCCAAACCCTGGATTTATGCCACATCCAACCTGGCTTCTGGAGTCCCTGTTCGC
41       S  S  P  K  P  W  I  Y  A  T  S  N  L  A  S  G  V  P  V  R

               190       200       210       220       230       240
181     TTCAGTGGCAGTGGGTCTGGGACCTCTTACTCTCTCACAATCAGTAGAGTGGAGGCTGAA
61       F  S  G  S  G  S  G  T  S  Y  S  L  T  I  S  R  V  E  A  E

               250       260       270       280       290       300
241     GATGCTGCCACTTATTACTGCCAGCAGTGGACTAGTAACCCACCCACGTTCGGTGGTGGG
81       D  A  A  T  Y  Y  C  Q  Q  W  T  S  N  P  P  T  F  G  G  G

               310       320
301     ACCAAGCTGGAGATCAAA
101      T  K  L  E  I  K
```

Fig.6

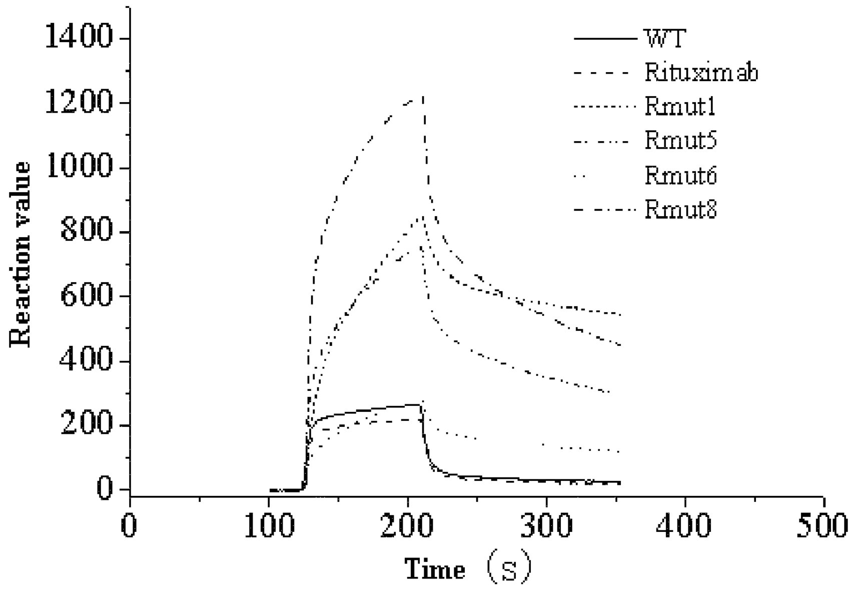


Fig. 7

CTLA-4 **extramembrane fragment**

ATG CAC GTG GCC CAG CCT GCT GTG GTA CTG GCC AGC AGC CGA GGC ATC GCC AGC TTT GTG
M H V A Q P A V V L A S S R G I A S F V

31
TGT GAG TAT GCA TCT CCA GGC AAA GCC ACT GAG GTC CGG GTG ACA GTG CTT CGG CAG GCT
C E Y A S P G K A T E V R V T V L R Q A

53 55
GAC AGC CAG GTG ACT GAA GTC TGT GCG GCA ACC TAC ATG ATG GGG AAT GAG TTG ACC TTC
D S Q V T E V C A A T Y M M G N E L T F

63
CTA GAT GAT TCC ATC TGC ACG GGC ACC TCC AGT GGA AAT CAA GTG AAC CTC ACT ATC CAA
L D D S I C T G T S S G N Q V N L T I Q

97
GGA CTG AGG GCC ATG GAC ACG GGA CTC TAC ATC TGC AAG GTG GAG CTC ATG TAC CCA CCG
G L R A M D T G L Y I C K V E L M **Y** P P

106
CCA TAC TAC CTG GGC ATA GGC AAC GGA ACC CAG ATT TAT GTA ATT GAT CCA GAA CCG TGC
P Y Y L G I G N G T Q I Y V I D P E P C

CCA GAT TCT GAT CAG
P D S D Q

Fig. 8

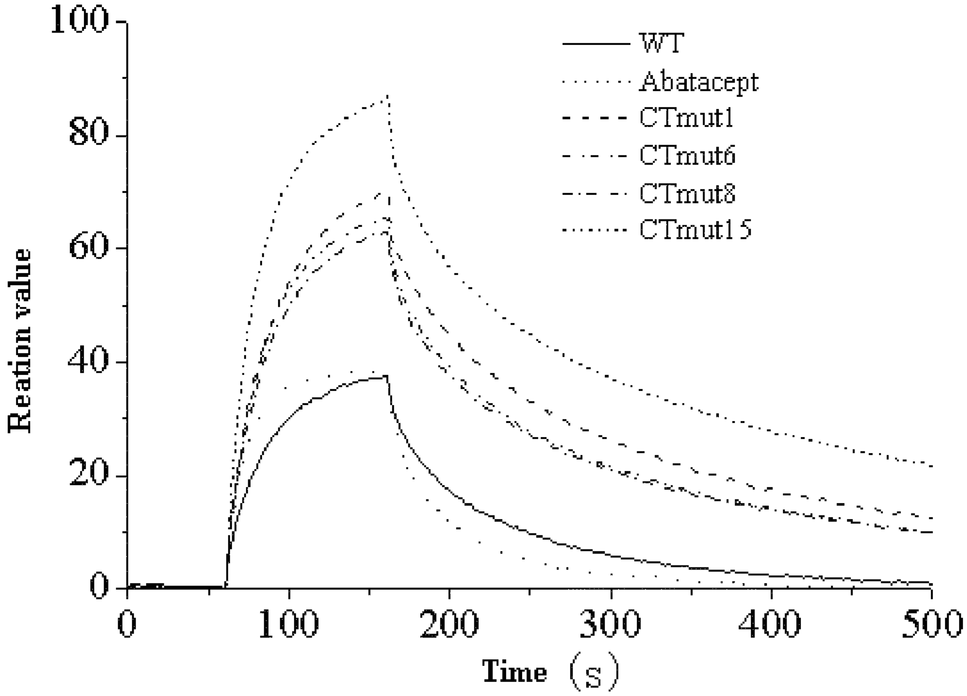
100
80
60
40
20
0
Reation value
0
100
200
300
400
500
Time (s)
WT
Abatacept
CTmut1
CTmut6
CTmut8
CTmut15

Fig. 9

## INTERNATIONAL SEARCH REPORT

International application No.
PCT/CN2009/001079

**A. CLASSIFICATION OF SUBJECT MATTER**

See extra sheet

According to International Patent Classification (IPC) or to both national classification and IPC

B. FIELDS SEARCHED

Minimum documentation searched (classification system followed by classification symbols)

IPC: G06F; C07K; C12N

Documentation searched other than minimum documentation to the extent that such documents are included in the fields searched

Electronic data base consulted during the international search (name of data base and, where practicable, search terms used)

See extra sheet

C. DOCUMENTS CONSIDERED TO BE RELEVANT

| Category* | Citation of document, with indication, where appropriate, of the relevant passages | Relevant to claim No. |
|---|---|---|
| A | CN101228270 A (BAYER SCHERING PHARMA AG) 23 July 2008 (23.07.2008) see the whole document, especially page 42, line 8 –page 43, line 7 of the description, and the abstract | 1-5 |
| A | CN101496014 A (MEDICAL RES COUNCIL) 29 July 2009 (29.07.2009) see the whole document, especially page 10, line 17 –page 12, line 5 of the description, and the abstract | 1-5 |
| A | CN101501692 A (MEDICAL RES COUNCIL) 05 August 2009 (05.08.2009) see the whole document, especially page 16, line 6-page 20, line 24 of the description, and the abstract | 1-5 |

☐ Further documents are listed in the continuation of Box C. ☒ See patent family annex.

* Special categories of cited documents:

"A" document defining the general state of the art which is not considered to be of particular relevance

"E" earlier application or patent but published on or after the international filing date

"L" document which may throw doubts on priority claim (S) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&"document member of the same patent family

| Date of the actual completion of the international search | Date of mailing of the international search report |
|---|---|
| 14 April 2010（14.04.2010） | **13 May 2010 (13.05.2010)** |

Name and mailing address of the ISA/CN
The State Intellectual Property Office, the P.R.China
6 Xitucheng Rd., Jimen Bridge, Haidian District, Beijing, China
100088
Facsimile No. 86-10-62019451

Authorized officer
WU, Yongqing
Telephone No. (86-10)62411116

**INTERNATIONAL SEARCH REPORT**
Information on patent family members

International application No.
PCT/CN2009/001079

| Patent Documents referred in the Report | Publication Date | Patent Family | Publication Date |
|---|---|---|---|
| CN101228270 A | 23.07.2008 | WO2007010285 A2 | 25. 01.2007 |
| | | EP1904629 A2 | 02. 04.2008 |
| | | INDELNP200801406 E | 08. 08.2008 |
| | | KR20080036583 A | 28. 04.2008 |
| | | JP2009504141 T | 05. 02.2009 |
| | | CA2615753 A1 | 25. 01.2007 |
| | | US2009275047 A1 | 05. 11.2009 |
| CN101496014 A | 29.07.2009 | WO2007141516 A2 | 13. 12.2007 |
| | | WO2007141516 A3 | 19. 06.2008 |
| | | EP2035983 A2 | 18. 03.2009 |
| | | US2009265114 A1 | 22. 10.2009 |
| | | JP2009539365 T | 19. 11.2009 |
| CN101501692 A | 05.08.2009 | GB2440736 A | 13. 02.2008 |
| | | WO2008017863 A2 | 14. 02.2008 |
| | | WO2008017863 A3 | 03. 07.2008 |
| | | EP2050027 A2 | 22. 04.2009 |
| | | JP2010500543 T | 07. 01.2010 |

Form PCT/ISA /210 (patent family annex) (July 2009)

## INTERNATIONAL SEARCH REPORT

International application No.
PCT/CN2009/001079

Continuation of: CLASSIFICATION OF SUBJECT MATTER

G06F 17/50 (2006.01) i
C07K 16/00 (2006.01) i
C07K 14/00 (2006.01) i

Continuation of: Electronic data base consulted during the international search (name of data base and, where practicable, search terms used)

WPI; EPODOC; PUBMED; CNPAT; CNKI and keywords: PROTEIN, ANTIBODY, ROOT MEAN SQUARE DEVIATION, CRYSTAL, COCRYSTAL, ENERGY

Form PCT/ISA /210 (extra sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6399061 B


### Non-patent literature cited in the description


- **Clark L A ; Ganesan S ; Papp S et al.** Trends in antibody sequence changes during the somatic hypermutation process.[J]. *J Immunol.,* 2006, vol. 177 (1), 333-340
- **Lo C L ; Chothia C ; Janin J.** The atomic structure of protein-protein recognition sites.[J]. *J Mol Biol.,* 1999, vol. 285 (5), 2177-2198
- **Berman, Westbrook et al.** *Nucleic Acids Res.,* 2000, vol. 28, 235-242, www.pdb.org
- **Pnina D O.** Structure and energetics of ligand binding to proteins: Escherichia coli dihydrofolate reductase-trimethoprim, a drug-receptor system[J]. *Proteins: Structure, Function, and Genetics,* 1988, vol. 4 (1), 31-47
- **Dunbrack R L.** Rotamer Libraries in the 21st Century. *Current Opinion in Structural Biology,* 2002, vol. 12 (4), 431-440
- **Ponder J W ; Richards F M.** Tertiary templates for proteins : Use of packing criteria in the enumeration of allowed sequences for different structural classes[J]. *Journal of Molecular Biology,* 1987, vol. 193 (4), 775-791
- **Ding H Q ; Karasawa N ; Goddard I I.** Atomic level simulations on a million particles: The cell multipole method for Coulomb and London nonbond interactions. *J. Chem. Phys.,* 1992, vol. 97 (6), 4309-4315
- **Senderowitz H ; Guarnieri F ; Still W C.** A Smart Monte Carlo Technique for Free Energy Simulations of Multiconformational Molecules. Direct Calculations of the Conformational Populations of Organic Molecules [J]. *J. Am. Chem. Soc.,* vol. 117 (31), 8211-8219
- **Bernard, R. B. ; E. B. Robert et al.** CHARMM: A program for macromolecular energy, minimization, and dynamics calculations. *J Comput Chem.,* 1983, vol. 4 (2), 187-217
- **Becker, O. M. ; M. Karplus.** *Guide to Biomolecular Simulations,* 2005
- **Im, W. ; Lee, M. S. ; Brooks, C. L.** Generalized born model with a simple smoothing function. *J. Comput. Chem.,* 2003, vol. 24, 1691-1702
- **Kuhn, B. ; Gerber, P. ; Schulz-Gasch, T. ; Stahl, M.** Validation and use of the MM-PBSA approach for drug discovery. *J. Med. Chem.,* 2005, vol. 48, 4040-4048
- **Alonso, H. ; Bliznyuk, A. A. ; Gready, J. E.** Combining docking and molecular dynamic simulations in drug design. *Med. Res. Rev.,* 2006, vol. 26, 531-568
- **Fogolari, F. ; A. Brigo et al.** protocol for MM/PBSA molecular dynamics simulations of proteins. *Biophys J,* 2003, vol. 85 (1), 159-66
- **Chong L T ; Duan Y ; Wang L et al.** Molecular dynamics and free-energy calculations applied to affinity maturation in antibody 48G7.[J]. *Proc Natl Acad Sci U S A.,* 1999, vol. 96 (25), 14330-14335
- **Novotny J ; Sharp K.** Electrostatic fields in antibodies and antibody/ antigen complexes.[J]. *Prog Biophys Mol Biol.,* 1992, vol. 58 (3), 203-224
- **Novotny J ; Bruccoleri R E ; Davis M et al.** Empirical free energy calculations: a blind test and further improvements to the method.[J]. *JMol Biol.,* 1997, vol. 268 ((2)), 401-411
- **Ding, H. Q. ; N. Karasawa et al.** Atomic level simulations on a million particles: The cell multipole method for Coulomb and London nonbond interactions. *J. Chem. Phys.,* 1992, vol. 97 (6), 4309-4315
- **Hieter PA ; Max EE ; Seidman JG ; Maizel JV Jr ; Leder P.** *Cell.,* November 1980, vol. 22, 197-207
- **Ellison JW ; Berson BJ ; Hood LE.** *Nucleic Acids Res.,* 10 July 1982, vol. 10 (13), 4071-9
- **Carter, P. ; L. Presta et al.** Humanization of an anti-p185HER2 antibody for human cancer therapy. *Proc Natl Acad Sci U S A,* 1992, vol. 89 (10), 4285-9
- **Carter P et al.** Humanization of an anti-p185HER2 antibody for human cancer therapy. *Pro Natl Acad Sci USA,* 1992, vol. 89, 4285-4289
- **Friguet B ; Chaffotte AF ; Djavadi-Ohaniance L ; Goldberg ME.** Measurements of the true affinity constant in solution of antigen-antibody complexes by enzyme-linked immunosorbent assay. *J Immunol Methods,* 1985, vol. 77, 305-319

- **Hieter PA ; Max EE ; Seidman JG ; Maizel JV Jr ; Leder P.** *Cell,* November 1980, vol. 22, 197-207
- **Du, J. ; Wang, H. ; Zhong, C.** Structural Basis for Recognition of CD20 by Therapeutic Antibody Rituximab. *J Biol Chem,* 2007, vol. 282 (20), 15073-15080